# NEUE EUROPÄISCHE PATENTSCHRIFT

(11) **EP 1 633 471 B2**
(45) Veröffentlichungstag und Bekanntmachung des Hinweises auf die Entscheidung über den Einspruch: **18.10.2017**
(45) Hinweis auf die Patenterteilung: 25.03.2009
(21) Anmeldenummer: 04739697.3
(22) Anmeldetag: 08.06.2004
(51) Int. Cl.: B01J 13/02, B01J 13/04, B01J 13/22, C11D 3/50

(54) **LAGERSTABILES POLYELEKTROLYTKAPSELSYSTEM AUF BASIS VON PEROXYCARBONSÄUREN**
PERCARBOXYLIC ACID-BASED POLYELECTROLYTE CAPSULE SYSTEM HAVING A LONG SHELF LIFE
SYSTEME DE CAPSULE POLYELECTROLYTIQUE A BASE D'ACIDES PEROXYCARBONIQUES PRESENTANT UNE BONNE STABILITE AU STOCKAGE

(30) Priorität: 13.06.2003 DE 10327127; 22.12.2003 DE 10361170
(43) Veröffentlichungstag der Anmeldung: 15.03.2006
(73) Patentinhaber: Henkel AG & Co. KGaA, 40589 Düsseldorf (DE)
(72) Erfinder: SCHMIEDEL, Peter, 40599 Düsseldorf (DE); BUZZACCHI, Matteo, Department of Physics, Bath BA2 7AY (GB); KAISER, Heribert, 40223 Düsseldorf (DE); VON RYBINSKI, Wolfgang, 40593 Düsseldorf (DE); ORLICH, Bernhard, 08005 Barcelona (ES)
(86) Internationale Anmeldenummer: PCT/EP2004/006169
(87) Internationale Veröffentlichungsnummer: WO 2004/110613

(56) Entgegenhaltungen:
- EP-A- 0 272 402
- EP-A- 0 435 379
- EP-A2- 0 390 287
- WO-A-03/002248
- WO-A1-00/03797
- WO-A1-00/77281
- WO-A1-01/01926
- WO-A1-00//17311
- WO-A1-97//14780
- CA-A1- 2 326 560
- DE-A1- 10 100 689
- US-A- 3 908 045
- US-A- 4 126 573
- US-A- 4 225 451
- US-A- 5 246 620
- US-A- 5 279 757
- US-A- 5 419 846
- US-A1- 2003 219 384
- G.REINHARDT: 'Imidoperoxicarbonsäuren als potentielle Bleichmittel für' SÖFW JOURNAL Bd. 120, Nr. 7, 1994, Seiten 411 - 416
- GERO DECHER: 'Fuzzy Nanoassemblies: Toward Layered Polymeric Multicomposites' SCIENCE Bd. 277, Nr. 1232, 1997, Seiten 1232 - 1237

## Beschreibung

Die vorliegende Erfindung betrifft ein Verfahren zur Herstellung eines mit mindestens einer organischen Peroxycarbonsäure beladenen mehrschichtigen Kapselsystems sowie das auf diese Weise hergestellte Kapselsystem selbst. Weiterhin betrifft die vorliegende Erfindung die Verwendung dieses Kapselsystems als Bleichmittel bzw. Bleichmittelkomponente, insbesondere für seinen Einsatz in Wasch- und Reinigungsmitteln, insbesondere in flüssigen Wasch- und Reinigungsmitteln, Zahnpflegemitteln, Haarfärbemitteln und Entfärbungs- bzw. Bleichmittelzusammensetzungen für technische Anwendungen sowie die dieses Kapselsystem enthaltenen Produkte selbst, d. h. Wasch- und Reinigungsmittel, insbesondere flüssige Wasch- und Reinigungsmittel, Zahnpflegemittel, Haarfärbemittel und Entfärbungs- bzw. Bleichmittelzusammensetzungen für technische Anwendungen, welche das erfindungsgemäße Kapselsystem enthalten.

Bei flüssigen, insbesondere wasserhaltigen Wasch- und Reinigungsmitteln, die sich aufgrund ihrer positiven Produkteigenschaften, wie einer besseren und schnelleren Löslichkeit und Anwendbarkeit, einer zunehmenden Beliebtheit erfreuen, ist die Einformulierung bzw. Inkorporierung von Bleich(mittel)komponenten aus zahlreichen Gründen problematisch. So verlieren eingesetzte Bleichmittel beispielsweise aufgrund von Zersetzungs- bzw. Hydrolysereaktionen und Inkompatibilitäten gegenüber anderen Bestandteilen der Waschmittelformulierung, wie z. B. Enzymen oder Tensiden, häufig schon bei der Lagerung ihre Aktivität. Als nachteilige Folge verliert die Waschmittelformulierung hierdurch deutlich an Waschleistung, insbesondere Bleichvermögen, so daß insbesondere bleichbare Verschmutzungen nicht mehr zufriedenstellend entfernt werden können.

Die üblicherweise für feste Waschmittelformulierungen verwendeten Bleichmittelkomponenten, wie beispielsweise Perborate oder Percarbonate, sind äußerst feuchtigkeitsempfindlich, so daß in einem flüssigen und insbesondere wasserhaltigen Wasch- und Reinigungsmittel aufgrund des Verlustes von Aktivsauerstoff häufig innerhalb weniger Tage ein deutlicher Rückgang ihrer Bleichwirkung zu beobachten ist. Daher können derartige Wirkstoffe zum Zeitpunkt ihrer Anwendung, insbesondere in der Waschflotte, häufig ihre Bleichwirkung bereits verloren haben und somit unwirksam sein.

Demgegenüber sind Peroxycarbonsäuren, insbesondere Imidoperoxycarbonsäuren, deren wichtigster Vertreter Phthalimidocapronsäure (PAP) ist, effizienter und weniger hydrolyseempfindlich und im Stand der Technik als Bleichmittel für Wasch- und Reinigungsmittel bekannt. Jedoch ist ihre Lagerstabilität bei weitem nicht ausreichend, um eine langfristige Einsetzbarkeit des entsprechenden Wasch- oder Reinigungsmittels ohne einhergehenden Aktivitätsverlust zu gewährleisten. Besonders problematisch ist daher der Einsatz von Peroxycarbonsäuren, insbesondere Imidoperoxycarbonsäuren, in flüssigen Wasch- und Reinigungsmitteln.

Aufgrund der Nachteile, die sich in bezug auf eine Veränderung der Wasch- oder Reinigungsmittelformulierung infolge des Abbaus von Imidoperoxycarbonsäuren, insbesondere PAP, ergeben, wurde im Stand der Technik versucht, die Imidoperoxycarbonsäuren (z. B. PAP) wirkungsvoll zu verkapseln, so daß die Imidoperoxycarbonsäure mit ihrer Umgebung, insbesondere den übrigen Komponenten der Wasch- bzw. Reinigungsmittelformulierung, nicht in direkten Kontakt geraten kann bzw. der Kontakt der Imidoperoxycarbonsäuren mit ihrer Umgebung reduziert wird.

So werden im Stand der Technik häufig Wachse als Schutzhülle für empfindliche Waschmittelzusatzstoffe, wie beispielsweise Peroxycarbonsäuren, verwendet. In diesem Zusammenhang beschreibt die EP 0 510 761 B1 bzw. die zu derselben Patentfamilie gehörende US-A-5 230 822 ein allgemeines Verfahren zur Verkapselung von Waschmittelzusatzstoffen beliebiger Art, wie z. B. Enzymen, Bleichmitteln, unter anderem auch PAP, Bleichmittelvorläufern und Bleichkatalysatoren. Die dort beschriebene Verwendung eines Wachses, dessen Schmelzpunkt zwischen 40 °C und 50 °C liegt, führt dazu, daß die Wirksubstanzen in der Waschflotte erst bei Temperaturen oberhalb des Schmelzpunktes des verwendeten Wachses freigesetzt werden, was vor dem Hintergrund der Entwicklung leistungsfähiger Wasch- und Reinigungsmittelformulierungen und der Einsparung von Energiekosten nachteilig ist, da auch bei etwa 30 °C gewaschen werden soll. Weiterhin weist die Verwendung eines Wachses mit einem hohen Schmelzpunkt den Nachteil auf, daß es insbesondere bei niedrigen Temperaturen Rückstände auf der Wäsche verursacht, da es bei diesen Temperaturen nicht vollständig emulgiert wird.

Darüber hinaus wurde im Stand der Technik versucht, andere Methoden zur Stabilisierung von Peroxycarbonsäuren zu entwickeln: So beschreibt die US-A-6 080 715 Granulatzusammensetzungen auf Basis von Phthalimidoperoxycapronsäure. Die dort beschriebenen Granulate umfassen Phthalimidoperoxycapronsäure (PAP), N-Oxidverbindungen von tertiären Aminen und Carbonsäuren mit einem pKₐ-Wert kleiner als 3,5. Die dort beschriebenen Granulate liegen nicht in Form von Kapselsystemen aus einem Kern und einer Hülle, sondern lediglich in Form einer Mischung der jeweiligen Komponenten vor. Hieraus folgt, daß die Peroxycarbonsäure insbesondere im Bereich der Oberfläche der Granulate in direktem Kontakt mit der Umgebung stehen kann, was hinsichtlich ihrer Stabilität nachteilig ist. Weiterhin sind diese Granulate nicht für einen Einsatz in wäßrigen Dispersionen, insbesondere flüssigen Wasch- und Reinigungsmitteln, vorgesehen. Vielmehr ist in diesem Dokument lediglich der Einsatz der Granulate in pulverförmigen Wasch- und Reinigungsformulierungen vorgesehen.

Die WO 97/39097 A1 beschreibt Bleichmittelgranulate, welche als innige Mischung von Peroxycarbonsäure, beispielsweise PAP, und Tensid, beispielsweise einem Aminoxid, vorliegt. Die dort beschriebenen Granulate weisen ebenfalls keine Kapselstruktur aus einem Kern und einer Kapselhülle auf. Die Granulate sind dabei vorrangig in bezug auf eine Verringerung von durch das Bleichmittel hervorgerufenen Beschädigungen der Textilfarbstoffe optimiert.

In der US-A-4 126 573 sind Bleichpartikel beschrieben, deren Kern Peroxycarbonsäuren enthält und deren nur einschichtige Hülle wasserlösliche Tenside, beispielsweise organische anionische Sulfat- oder Aminoxidverbindungen, umfaßt.

Weiterhin beschreibt die WO 94/15010 A1 die Beschichtung bzw. Verkapselung eines Wirkstoffs mit wasserlöslichen sauren Polymeren, beispielsweise Polyacrylsäure, wobei das Kapselsystem eine nur einschichtige Hülle umfaßt und als Wirkstoff ein Bleichaktivator vorgesehen ist.

In der WO 01/51196 A1 wird die Verkapselung von ungeladenen bzw. hydrophoben Substanzen mit mindestens einer Polyelektrolytschicht beschrieben. Bei den dort angeführten Substanzen bzw. Wirkstoffen handelt es sich beispielsweise um Vitamine, Hormone, Wachstumsfaktoren, Pestizide und Antibiotika. Der nichtgeladene bzw. hydrophobe Wirkstoff muß dabei zunächst mit einer amphiphilen Substanz beschichtet werden, und das Aufbringen der jeweiligen Polyelektrolytkapselhülle erfolgt über Adsorptionsprozesse aus einer Lösung heraus. Dabei liegen die Dicken der erhaltenen Schichten nur im molekularen Bereich, so daß die Wechselwirkung zwischen den einzelnen Schichten über ihre gesamte Dicke im wesentlichen über interionische Wechselwirkungen, die zur Ausbildung von Komplexen führt, erfolgt. Zwischen dem Aufbringen der einzelnen Schichten muß dabei zunächst das nichtadsorbierte Material entfernt werden. Dieser Prozeß ist aufwendig und großtechnisch wenig praktikabel. Aufgrund der nur geringen Hüllschichtdicken eignen sich diese Systeme nicht zur effizienten Stabilisierung von Peroxycarbonsäuren in wäßrigen Formulierungen mit gezielter Freisetzung in der Waschflotte.

Weiterhin beschreibt die WO 02/17888 A2 eine allgemeine Lehre zur Herstellung von Kapseln, welche eine Hüllschicht aus entgegengesetzt geladenen Polyelektrolyten aufweist, wobei die Kapseln mittels eines "Layer-by-Layer"-(LbL-)Verfahrens hergestellt werden. Die zu verkapselnde Substanz kann dabei beispielsweise Fluorescein sein. Weiterhin wird dort ein allgemeines Verfahren zur Beeinflussung der Permeabilität der dort beschriebenen Kapseln, insbesondere über Modifikationen des pH-Wertes oder durch Einfluß von Licht, beschrieben.

Wie bei dem zuvor beschriebenen Stand der Technik, so handelt es sich auch hierbei um Hüllschichten mit einer Dicke nur im molekularen Bereich, die über Adsorption aus einer Lösung - mit den damit verbundenen verfahrenstechnischen Nachteilen - aufgetragen werden. Aufgrund der nur geringen Hüllschichtdicken eignen sich diese Systeme ebenfalls nicht zur effizienten Stabilisierung von Peroxycarbonsäuren in wäßrigen Formulierungen mit gezielter Freisetzung in der Waschflotte.

Auch die WO 02/31092 A2 beschreibt ein Beschichtungs- bzw. Verkapselungsverfahren auf Basis der "Layer-by-Layer"-Technik, wobei hierbei nur flüssige Substanzen, beispielsweise Parfümöle, verkapselt werden.

Schließlich beschreibt die WO 99/47252 A2 allgemein die Herstellung von Nano- und Mikrokapseln durch schichtweise Polyelektrolyt-Selbstassemblierung. Auch hierbei handelt es sich um Kapseln, die eine nur geringe Wandstärke aufweisen, wobei die Hüllen in der Weise hergestellt werden, daß zu einer Dispersion von zu verkapselnden Templatpartikeln die entsprechenden Polyelektrolytmoleküle gegeben werden. Dabei ist zwischen dem Aufbringen der einzelnen Hüllen auch hier ein aufwendiges Entfernen der überschüssigen Polyelektrolytmoleküle erforderlich. Die Verkapselung von Peroxycarbonsäuren, einhergehend mit speziell hierfür erforderlichen Maßnahmen, ist in diesem Dokument nicht offenbart.

Vor diesem Hintergrund besteht somit eine Aufgabe der vorliegenden Erfindung darin, eine Einlagerung oder Verkapselung bzw. Beschichtung von Peroxycarbonsäuren, insbesondere Imidoperoxycarbonsäuren, wie Phthalimidoperoxycapronsäure (PAP), mit gegenüber dem Stand der Technik verbesserten Eigenschaften sowie ein entsprechendes Herstellungsverfahren bereitzustellen.

Eine weitere Aufgabe der vorliegenden Erfindung besteht darin, Peroxycarbonsäuren, insbesondere Imidoperoxycarbonsäuren, wie Phthalimidoperoxycapronsäure (PAP), in lagerstabiler Form bereitzustellen. Dabei soll eine Konfektionierungsform von Peroxycarbonsäuren entwickelt werden, die ein Auf- oder Anlösen der Peroxycarbonsäuren zumindest im wesentlichen verhindert oder verringert und vorzugsweise deren festen bzw. kristallinen Zustand auch in Gegenwart von Tensiden oder in einer sonstigen Umgebung, die ein Lösungsvermögen für Peroxycarbonsäuren besitzt, insbesondere in Wasch- oder Reinigungsmittelformulierungen, erhält, wobei insbesondere ein Kontakt der Peroxycarbonsäuren mit der Umgebung zumindest im wesentlichen verhindert bzw. zumindest verringert werden soll.

Noch eine weitere Aufgabe der vorliegenden Erfindung ist die Bereitstellung eines Kapselsystems, das mit Peroxycarbonsäuren in fester Form beladen ist und zu einer guten Stabilisierung der Peroxycarbonsäuren und damit zu einer verbesserten Lagerstabilität führt. Insbesondere soll im Rahmen der vorliegenden Erfindung ein Kapselsystem bereitgestellt werden, das insbesondere während des Waschvorganges weitgehend rückstandslos aufgelöst und/oder solubilisiert bzw. dispergiert wird, so daß die Freisetzung der Peroxycarbonsäuren nicht behindert wird und gleichzeitig Rückstände auf der Wäsche vermieden werden. Insbesondere soll ein solches Verfahren es ermöglichen, ein mit mindestens einer Peroxycarbonsäure als Wirksubstanz beladenes Kapselsystem herzustellen, welches die zuvor geschilderten Nachteile des Standes der Technik zumindest weitgehend vermeidet.

Die Anmelderin hat nun überraschenderweise herausgefunden, daß organische Peroxycarbonsäuren, wie Imidoperoxycarbonsäuren (z. B. PAP), durch ein mehrschichtiges Kapselsystem, dessen Schichten bzw. Hüllschichten Polyelektrolyte bzw. ionische Tenside umfassen oder hieraus bestehen, in hohem Maße stabilisiert werden können.

Gegenstand der vorliegenden Erfindung gemäß einem ersten Aspekt ist somit ein Verfahren gemäß Anspruch 1.

Im Rahmen der vorliegenden Erfindung wird die zu verkapselnde Peroxycarbonsäure in eine Kapselhülle, welche mindestens zwei Hüllschichten umfaßt, eingelagert bzw. von ihr umhüllt, so daß die Peroxycarbonsäure den Kern des erfindungsgemäßen Kapselsystems bildet; es liegt somit erfindungsgemäß ein Kapselsystem mit einer Kern/Hülle-Struktur vor. Das erfindungsgemäße Kapselsystem kann auch mehrere Kapselkerne umfassen, insbesondere wenn sich bei der Herstellung, beispielsweise in einer Wirbelschichtapparatur, Agglomerate aus Peroxycarbonsäureteilchen bilden. Auch können sich insbesondere nach Aufbringen der jeweiligen Hüllschichten bzw. der Kapselhülle Agglomerate bilden. Auf diese Weise kann sozusagen eine Matrix entstehen, in welche mehrere Kapselkerne eingebettet bzw. eingelagert sind.

Dabei sind die Hüllschichten bzw. Schichten der Kapselhülle von unterschiedlicher Natur und derart ausgebildet, daß eine erste Schicht (Hüllschicht) die Peroxycarbonsäure vollständig umhüllt bzw. beschichtet und die zweite sowie gegebenenfalls dritte, vierte etc. Hüllschicht bzw. Umhüllung in direktem Kontakt zu der jeweils benachbarten Umhüllung bzw. Hüllschicht steht. Dabei ist es erfindungsgemäß besonders bevorzugt, daß mindestens die erste bzw. innerste Hüllschicht (Umhüllung) zumindest im wesentlichen inert gegenüber der Peroxycarbonsäure ist, d. h. im wesentlichen nicht zu unerwünschten chemischen Reaktionen, wie insbesondere Abbau-, Oxidations- bzw. Reduktionsreaktionen und/oder Hydrolysereaktionen, mit der Peroxycarbonsäure führt.

Die weiteren Komponenten bzw. Hüllschichten, welche in dem erfindungsgemäßen Verfahren zur Herstellung des erfindungsgemäßen Kapselsystems verwendet werden, sollten vorzugsweise gleichermaßen derart ausgewählt sein, daß sie zumindest im wesentlichen kompatibel in bezug auf die zu verkapselnde Peroxycarbonsäure sind, d. h. es sollten keine unerwünschten chemischen Reaktionen, wie insbesondere Abbau-, Oxidations- bzw. Reduktionsreaktionen und/oder Hydrolysereaktionen, zwischen diesen Komponenten und der Peroxycarbonsäure und keine durch die weiteren Komponenten induzierten Reaktionen der Peroxycarbonsäure auftreten, welche zu ihrem Abbau, insbesondere Aktivitätsverlust, führen.

Die jeweiligen Hüllschichten sind dabei auf Basis von Polyelektrolyten und/oder ionischen Tensiden ausgestaltet, wobei im Rahmen der vorliegenden Erfindung der Begriff "auf Basis von Polyelektrolyten bzw. ionischen Tensiden" so zu verstehen ist, daß neben den Polyelektrolyten bzw. ionischen Tensiden auch noch weitere Materialien, insbesondere wie nachfolgend angeführt, zur Ausbildung der einzelnen Hüllschichten verwendet werden können.

Die Polyelektrolyte und/oder ionischen Tenside der jeweils unmittelbar aneinandergrenzenden Hüllschichten sind erfindungsgemäß entgegengesetzt geladen, wobei bei mehr als zwei Schichten, z. B. drei, vier, fünf etc. Schichten, die erste, dritte, fünfte etc. Schicht jeweils gleichgeladen oder vom gleichen ionischen Charakter, d. h. anionisch oder kationisch, sind und die zweite, vierte, sechste etc. Schicht jeweils gleichgeladen, aber entgegengesetzt zu der ersten, dritten, fünften etc. Schicht geladen sind. Somit resultiert erfindungsgemäß ein Kapselsystem mit mehrschichtiger Kapselhülle, wobei die jeweils unmittelbar benachbarten bzw. unmittelbar aufeinanderfolgenden bzw. unmittelbar aneinandergrenzenden Schichten (Umhüllungen bzw. Hüllschichten) entgegengesetzt geladen sind, d. h. sozusagen "gegenionisch" ausgebildet sind.

Im Rahmen der Beschreibung zu der vorliegenden Erfindung sind die Begriffe "Polyelektrolyt" bzw. "Polyion" im wesentlichen synonym zu verstehen. Hierunter kann insbesondere ein Polymer mit einer großen Zahl ionisch dissoziierbarer bzw. dissoziierter Gruppen, die z. B. integraler Bestandteil der Hauptkette des Polymers sein können oder aber an diese über Seitenketten angehängt sein können, verstanden werden. Im Rahmen der vorliegenden Erfindung weisen die verwendeten Polyelektrolyte bzw. Polyionen eine bestimmte, resultierende elektrische Nettoladung auf, so daß sie als Polyanionen, d. h. mit einer negativen Nettoladung versehen, oder als Polykationen, d. h. mit einer positiven Nettoladung versehen, vorliegen. Unter dem Begriff "Nettoladung" ist erfindungsgemäß zu verstehen, daß der Polyelektrolyt bzw. das Polyion überwiegend positiv bzw. überwiegend negativ geladene, dissoziierte Gruppen aufweist; dieser Begriff bezeichnet somit die resultierende Ladung des Polyelektrolyten bzw. des Polyions (d. h. ohne Gegenion). Die resultierende Gesamtladung einer Lösung bzw. Dispersion, die Polyelektrolyte, Gegenionen und Löse- bzw. Dispersionsmittel umfaßt, ist dagegen Null.

Im Rahmen des erfindungsgemäßen Verfahrens werden als Substanzen, die in das erfindungsgemäße Kapselsystem aus mehreren Hüllschichten eingeschlossen werden, organische Peroxycarbonsäuren verwendet. Diese können aus organischen Mono- und Diperoxycarbonsäuren ausgewählt sein. Beispiele hierfür sind insbesondere Dodekandiperoxysäure oder vorzugsweise Imidoperoxycarbonsäuren, besonders bevorzugt 6-Phthalimidoperoxycapronsäure (6-Phthalimidoperoxyhexansäure, PAP). Vorteilhafterweise sollte die Peroxycarbonsäure bei Atmosphärendruck einen Schmelzpunkt oberhalb von 25 °C, insbesondere oberhalb von 35 °C, vorzugsweise oberhalb von 45 °C, bevorzugt oberhalb von 50 °C, besonders bevorzugt oberhalb von 100 °C aufweisen. Hierdurch ist gewährleistet, daß die verwendete Peroxycarbonsäure zumindest im wesentlichen in Form fester Teilchen vorliegt, so daß ein Abbau der Peroxycarbonsäuren während der Herstellung des Kapselsystems im Rahmen des erfindungsgemäßen Verfahrens zumindest weitgehend vermieden bzw. verringert wird und bei Anwendung des erfindungsgemäßen Kapselsystems eine kontrollierte Freisetzung erreicht wird.

Dabei kann erfindungsgemäß ein Einstellen der Teilchengröße der Peroxycarbonsäuren beispielsweise vor dem Aufbringen der ersten Hüllschicht durch dem Fachmann als solche bekannte Verfahren durchgeführt werden, beispielsweise mittels Scherung, Vibration und/oder Ultraschalleintrag, Zerkleinern, Vermahlen etc., so daß erfindungsgemäß eine gezielte Anpassung der Teilchengröße entsprechend ihrer jeweiligen späteren Verwendung möglich ist.

Erfindungsgemäß bevorzugt weisen die gegebenenfalls mehreren Polyelektrolyte und/oder die gegebenenfalls mehreren ionischen Tenside einer Lösung dieselbe elektrische Nettoladung auf. Das Aufbringen der Lösung des mindestens einen Polyelektrolyten und/oder des mindestens einen ionischen Tensids kann in den Verfahrensschritten (a), (b) und oder (c) jeweils z. B. über Aufsprühen erfolgen, wobei für die erste Schicht ein Sprühtrockner und für die weiteren Schichten beispielsweise ein Wurstercoater eingesetzt werden kann. Es können aber auch alle Schichten in einer Wirbelschichtanlage aufgebracht werden. Der Ausdruck "dieselbe elektrische Nettoladung" kann als qualitative Angabe so verstanden werden, daß die oben angeführten Komponenten der Lösung zwar dasselbe Ladungsvorzeichen (positiv bzw. negativ) aufweisen, wobei aber gewisse Abweichungen von der Stöchiometrie möglich oder sogar gewünscht sein können, um beispielsweise eine gewisse Löslichkeit der Komponenten zu erhalten.

Der Kontakt bzw. die Haftung der einzelnen Hüllschichten aufeinander erfolgt im Bereich der von aneinandergrenzenden Hüllschichten ausgebildeten Grenzschichten- wie nachfolgend noch angeführt - insbesondere über elektrostatische Wechselwirkungen, wie Ion/Ion- bzw. Ion-/Dipol-Wechselwirkungen. Insbesondere können sich an der Grenze bzw. Grenzfläche zwischen zwei Hüllschichten aufgrund der entgegengesetzten Nettoladung der jeweiligen Hüllschichtmaterialien Komplexe (d. h. Polykation/Polyanion-Komplexe, Kationtensid/Polyanion-Komplexe, Polykation/Aniontensid-Komplexe, Aniontensid/Kationtensid-Komplexe, in Abhängigkeit von der jeweiligen Zusammensetzung der Hüllschichten) ausbilden, die einen guten Kontakt bzw. eine gute Haftung zwischen den einzelnen Hüllschichten gewährleisten. Darüber hinaus liegen andererseits aufgrund der relativ großen Dicken der einzelnen Hüllschichten in den jeweiligen Hüllschichten aber auch gewisse Teilbereiche vor, in bezug auf die keine Wechselwirkungen mit den benachbarten Hüllen bestehen.

In dem erfindungsgemäßen Verfahren weist der Polyelektrolyt bzw. das ionische Tensid der in Verfahrensschritt
(a) erhaltenen ersten Hüllschicht und der gegebenenfalls in Verfahrensschritt (c) erhaltenen dritten, fünften etc. Hüllschicht eine positive Nettoladung auf; dabei ist die erste Hüllschicht durch ein kationisches Tensid oder einen kationischen Polyelektrolyten (Polykation), vorzugsweise durch ein kationisches Tensid, gebildet, und der Polyelektrolyt der gegebenenfalls vorhandenen dritten, fünften etc. Hüllschicht ist ein kationischer Polyelektrolyt (Polykation). Der Polyelektrolyt bzw. das ionische Tensid der in Verfahrensschritt (b) erhaltenen zweiten Hüllschicht und der gegebenenfalls in Verfahrensschritt (c) erhaltenen vierten, sechsten etc. Hüllschicht weist dagegen eine negative Nettoladung auf und ist ein anionischer Polyelektrolyt (Polyanion). Die äußerste Hüllschicht, welche die Grenze zu einem Medium bzw. Milieu, d. h. zu der äußeren Umgebung, bildet, in welchem bzw. in welcher sich das Kapselsystem befinden kann, sollte vorzugsweise mindestens einen anionischen Polyelektrolyten aufweisen bzw. hieraus bestehen.

Erfindungsgemäß weist die erste Hüllschicht eine positive Nettoladung auf, so daß insbesondere aufgrund von elektrostatischen Wechselwirkungen, insbesondere von Ion/Ion- und/oder Ion/Dipol-Wechselwirkungen, ein guter Kontakt bzw. eine gute Haftung der ersten Hüllschicht in bezug auf die Peroxycarbonsäure gewährleistet wird. Diese Maßnahme wird vor dem Hintergrund durchgeführt, daß die Peroxycarbonsäure im Bereich der erfindungsgemäß bevorzugten pH-Werte eine negative (partiale) Oberflächenladung, insbesondere eine negative Partialladung, an der Oberfläche aufweist, die maßgeblich durch die Sauerstoffatome der Peroxycarboxylgruppe bedingt sind.

Bei den nach dem erfindungsgemäßen Verfahren hergestellten Kapselsystem umfaßt die erste Hüllschicht vorzugsweiset mindestens ein kationisches Tensid. Die zweite Hüllschicht und die gegebenenfalls dritte, vierte etc. Hüllschicht umfassen vorzugsweise bzw. bestehen vorzugsweise aus mindestens einem Polyelektrolyten oder einer Mischung aus mindestens einem Polyelektrolyten mit mindestens einem ionischen Tensid, wobei in einer solchen Mischung der mindestens eine Polyelektrolyt und das mindestens eine Tensid jeweils eine gleichartige Nettoladung aufweisen. Erfindungsgemäß umfaßt die zweite Hüllschicht und die gegebenenfalls dritte, vierte etc. Hüllschicht besonders bevorzugt mindestens einen Polyelektrolyten.

Das im Rahmen der vorliegenden Erfindung insbesondere für die erste Hüllschicht verwendete kationische Tensid kann ausgewählt sein aus quaternären Ammoniumsalzen, deren Ammoniumrest der allgemeinen Formel R¹R²R³N⁺ entspricht, wobei die Reste R¹, R² und R³, identisch oder verschieden, ein Wasserstoffatom oder einen linearen oder verzweigten Alkyl-, Alkylen- oder Alkinylrest mit 1 bis 40 Kohlenstoffatomen, insbesondere 1 bis 25 Kohlenstoffatomen, darstellen, wobei das Gegenion vorzugsweise halogenidfrei ist (z. B. Phosphat, Sulfat, Methylsulfat, Tosylat, Cumolsulfonat etc.). Das kationische Tensid kann ausgewählt sein aus der Gruppe von Alkyldimethylammoniumtensiden, N-Alkylpyridiniumsalzen und Esterquats. Erfindungsgemäß sollte das kationische Tensid keine Halogenidionen, insbesondere keine Chloridionen, enthalten oder zumindest im wesentlichen halogenidfrei, insbesondere chloridfrei, sein. Dabei kann das kationische Tensid ausgewählt sein aus Methylsulfat-, Sulfat-, Phosphat-, Tosylat- oder Cumolsulfonatverbindungen.

Der erfindungsgemäß insbesondere für die erste Hüllschicht verwendete kationische Polyelektrolyt sollte mindestens eine funktionelle Gruppe, ausgewählt aus quaternären Amin-, Imin- und Imidazolgruppen umfassen. Weiterhin kann der insbesondere für die erste Hüllschicht verwendete kationische Polyelektrolyt ausgewählt sein aus Aminoxiden oder Pyridin-N-oxiden, wie vorzugsweise Polyvinylpyridin-N-oxid, welche bei sauren pH-Werten protoniert vorliegen und daher kationisch sind. Dabei sollte der kationische Polyelektrolyt keine Halogenidionen, insbesondere keine Chloridionen, enthalten bzw. zumindest im wesentlichen halogenidfrei, insbesondere chloridfrei, sein.

Erfindungsgemäß kann für die erste Hüllschicht beispielsweise ein kationisches Tensid oder ein kationischer Polyelektrolyt verwendet werden, wie er z. B. von kosmetischen Anwendungen bekannt ist, z. B. mit der allgemeinen Bezeichnung "Polyquaternium^{®}", beispielsweise Polyquaternium-11^{®}, erhältlich unter dem Handelsnamen Luviquat^{®} PQ 11 (quaterniertes Vinylpyrrolidon/Dimethylaminomethylmethacrylat-Copolymer), sowie Polyquaternium-16^{®}, erhältlich unter dem Handelsnamen Luviquat^{®} FC 370 (Vinylpyrrolidon/quaterniertes Vinylimidazol-Copolymer).

Im Rahmen des erfindungsgemäßen Verfahrens kann der anionische Polyelektrolyt, insbesondere der zweiten, vierten etc. Hüllschicht, ein synthetischer anionischer Polyelektrolyt sein, der ausgewählt sein kann aus der Gruppe von polymeren Sulfonsäuren, insbesondere Polystyrolsulfonsäuren und deren (partiellen) Salzen; vorzugsweise Polycarbonsäuren und deren (partiellen) Salzen, wie insbesondere Polyacrylsäure, Polymaleinsäure und deren Copolymeren. Der anionische Polyelektrolyt kann z. B. ein polymeranalog sulfoniertes Polymer und/oder ein natürliches anionisches Polymer sein, das ausgewählt sein kann aus der Gruppe von Alginsäure, Xanthan bzw. derivatisierten natürlichen Polymeren, wie Carboxymethylcellulose.

Der erfindungsgemäß verwendete anionische Polyelektrolyt der zweiten, vierten etc. Hüllschicht kann aber z. B. auch ein natürliches anionisches Polymer sein, das beispielsweise ausgewählt sein kann aus der Gruppe von Alginat, Carboxymethylamylose, Carboxymethylcellulose, Carboxymethyldextran, Carageenan, Cellulosesulfat, Chondroitinsulfat, Chitosansulfat, Dextransulfat, Gummi Arabicum, Gummi Guar, Gummi Gellan, Heparin, Hyaluronsäure, Pektin, Xanthan und anionischen Proteinen. Der anionische Polyelektrolyt kann erfindungsgemäß aber auch ein synthetischer anionischer Polyelektrolyt sein und insbesondere ausgewählt sein aus der Gruppe von Polyacrylaten, anionischen Polyaminosäuren und deren Copolymeren, Polymaleinat, Polymethacrylat, Polystyrolsulfat, Polystyrolsulfonat, Polyvinylphosphat, Polyvinylphosphonat, Polyvinylsulfat, Polyacrylamidmethylpropansulfonat, Polylactat, Poly-(Butadien/Maleinat), Poly-(Ethylen/Maleinat), Poly-(Ethacrylat/Acrylat) und Poly-(Glycerin/Methacrylat).

Der in dem erfindungsgemäßen Verfahren verwendete kationische Polyelektrolyt, insbesondere der dritten, fünften etc. Hüllschicht, kann z. B. ein natürlicher kationischer Polyelektrolyt oder modifizierter, natürlicher kationischer Polyelektrolyt sein, der ausgewählt sein kann aus der Gruppe von Chitosan, modifizierten Dextranen, wie diethylaminoethylmodifizierten Dextranen, Hydroxymethylcellulosetrimethylamin, Lysozym, Polylysin, Protaminsulfat, Hydroxyethylcellulosetrimethylamin und kationischen Proteinen. Der kationische Polyelektrolyt kann ein synthetischer kationischer Polyelektrolyt sein und kann insbesondere ausgewählt sein aus der Gruppe von Polyallylamin, Polyallylaminhydrosalzen, Polyaminen, Polyvinylbenzyltrimethylammoniumsalzen, Polybren, Polydiallyldimethylammoniumsalzen, Polyethylenimin, Polyimidazolin, Polyvinylamin, Polyvinylpyridin, Poly-(Acrylamid/Methacryloxypropyltrimethylammoniumsalzen), Poly-(Diallyldimethylammoniumsalzen/N-Isopropylacrylamid), Poly-(Dimethylaminoethylacrylat/Acrylamid), Polydimethylaminoethylmethacrylat, Polydimethylaminoepichlorhydrin, Polyethyleniminoepichlorhydrin, Polymethacryloxyethyltrimethylammoniumsalzen, Hydroxypropylmethacryloxyethyldimethylammoniumsalzen, Poly-(Methyldiethylaminoethylmethacrylat/Acrylamid), Poly-(Methyl/Guanidin), Polymethylvinylpyridiniumsalzen, Poly-(Vinylpyrrolidon/Dimethylaminoethylmethacrylat) und Polyvinylmethylpyridiniumsalzen. Dabei sollte der kationische Polyelektrolyt im wesentlichen keine Halogenidionen, insbesondere keine Chloridionen, enthalten oder zumindest im wesentlichen halogenidfrei, insbesondere chloridfrei sein.

Gemäß einer erfindungsgemäß bevorzugten Ausführungsform kann im Rahmen des erfindungsgemäßen Verfahrens das kationische Tensid bzw. der kationische Polyelektrolyt, insbesondere der ersten, dritten, fünften etc. Hüllschicht, ein von dem pH-Wert, insbesondere von dem pH-Wert des Dispersionsmittels, abhängiges kationisches Tensid bzw. Polyelektrolyt sein. Dabei sollte das von dem pH-Wert abhängige kationische Tensid bzw. Polyelektrolyt bei einem sauren pH-Wert, bevorzugt bei einem pH-Wert kleiner als 7, insbesondere bei einem pH-Wert kleiner als 6,5, vorzugsweise kleiner als 6, zumindest im wesentlichen protoniert bzw. kationisch vorliegen. Das von dem pH-Wert abhängige kationische Tensid bzw. Polyelektrolyt kann bei einem neutralen oder alkalischen pH-Wert zumindest im wesentlichen deprotoniert bzw. elektrisch neutral vorliegen. Das von dem pH-Wert abhängige kationische Tensid bzw. Polyelektrolyt kann im Rahmen der vorliegenden Erfindung mindestens eine funktionelle Gruppe aufweisen, die ausgewählt sein kann aus Amino-, Imino-, Aminoxid-, Phosphinoxid- und Pyridin-N-oxidgruppen, vorzugsweise Aminoxid-, Phosphinoxid-, Pyridin-N-oxid- und Pyridiniumgruppen.

Wie zuvor erläutert, können bei dem erfindungsgemäßen Verfahren in zwischen den jeweiligen Hüllschichten ausgebildeten Grenzschichten Polykation/Polyanion-Komplexe, Kationtensid/Polyanion-Komplexe, Polykation/Aniontensid-Komplexe oder Aniontensid/Kationtensid-Komplexe ausgebildet werden (je nach jeweiliger Zusammensetzung der Hüllschichten). Dabei können diese Grenzschicht-Komplexe wasserlöslich, mäßig wasserlöslich oder vorzugsweise wasserunlöslich ausgebildet sein, wobei die Löslichkeit insbesondere von der Stöchiometrie abhängen kann. Unter dem Begriff "Stöchiometrie" wird in diesem Zusammenhang das Verhältnis der dissoziierbaren bzw. dissoziierten, gegebenenfalls elektrisch geladenen Gruppen bzw. Funktionen der kationischen Polyelektrolyte bzw. kationischen Tenside zu den entsprechenden Gruppen bzw. Funktionen der verwendeten anionischen Polyelektrolyte bzw. anionischen Tenside verstanden. Eine wasserunlösliche Ausbildung der Grenzschicht kann dabei insbesondere bei Ladungsneutralisation erreicht werden. Gemäß einer erfindungsgemäß bevorzugten Ausführungsform kann die Wasserlöslichkeit der Grenzschicht-Komplexe - wie oben angeführt - über den pH-Wert, insbesondere über den pH-Wert des Dispersionsmittel, gesteuert werden. Dies kann im Rahmen der vorliegenden Erfindung sowohl die konzentrierte Wasch- oder Reinigungsmittelsformulierung sein oder aber die beim Wasch- bzw. Reinigungsvorgang vorliegende verdünnte Waschflotte, d. h. insbesondere die verdünnte Wasch- oder Reinigungsmittelformulierung, sein.

Ohne sich auf eine spezielle Theorie festlegen zu wollen, verläuft die Ausbildung der Grenzschicht zwischen Hüllschichten -wie zuvor erläutert- beispielsweise aufgrund von chemischen und/oder physikalischen, insbesondere physikalischen Wechselwirkungen, insbesondere elektrostatischen Wechselwirkungen, wie beispielsweise interionischen Wechselwirkungen, wie Ion/Ion-Wechselwirkungen bzw. Ion/Dipol-Wechselwirkungen. Hieraus kann ein vorzugsweise wasserunlöslicher Komplex entstehen, der insbesondere in der konzentrierten Waschmittelformulierung die Stabilität der zu verkapselnden Peroxycarbonsäure erhöhen kann. Insbesondere weisen die Kapselhüllen - bedingt durch die Dicke der Hüllschichten - gleichermaßen Bereiche auf, in denen der ionische Polyelektrolyt bzw. das ionische Tensid vorzugsweise in nichtkomplexierter Form vorliegt, d. h. nicht mit entgegengesetzt geladenen Gruppen, insbesondere der benachbarten Hüllschicht, in Wechselwirkung steht. Demgegenüber sind die erfindungsgemäß verwendeten Polyelektrolyte bzw. Polyanionen und/oder Polykationen als solche wasserlöslich, so daß erfindungsgemäß eine Kapselhülle vorliegen kann, die alternierend vorzugsweise wasserunlösliche Schichten bzw.

Schichten, deren Wasserlöslichkeit beispielsweise von dem pH-Wert abhängt, umfassen, welche durch die vorgenannten Komplexe gebildet werden.

Durch den erfindungsgemäß bevorzugten Einsatz von kationischen Tensiden bzw. kationischen Polyelektrolyten, deren elektrische Ladung von dem pH-Wert abhängt, kann die Wasserlöslichkeit insbesondere der Grenzschicht wirkungsvoll beeinflußt werden. So liegt beispielsweise bei einem sauren pH-Wert, beispielsweise bei einem pH-Wert von etwa 3,5 bis etwa 5 (wie es für die erfindungsgemäße Wasch- oder Reinigungsformulierung der Fall sein kann), das von dem pH-Wert abhängige kationische Tensid bzw. der vom pH-Wert abhängige Polyelektrolyt vorzugsweise in protonierter bzw. positiv geladener, d. h. kationischer Form vor, so daß unter diesen Bedingungen die Wasserlöslichkeit der komplexierten Grenzschicht gering ist. Bei einer Erhöhung des pH-Wertes, wie es beispielsweise für den Einsatz einer erfindungsgemäßen Wasch- oder Reinigungsformulierung, die das erfindungsgemäße Kapselsystem enthält, in einer Waschflotte der Fall ist (beispielsweise Erhöhung auf einen pH-Wert von etwa 7 oder mehr), kann das eingesetzte, von dem pH-Wert abhängige kationische Tensid bzw. der vom pH-Wert abhängige Polyelektrolyt vorzugsweise in neutraler bzw. deprotonierter Form vorliegen, wodurch die Wasserlöslichkeit der jeweiligen Grenzschicht erhöht sein kann, so daß die Freisetzung der verkapselten Peroxycarbonsäure im Rahmen der Waschflotte hierdurch erleichtert werden kann und die Bildung von Rückständen vermieden werden kann.

Erfindungsgemäß besitzt das vom pH-Wert abhängige kationische Tensid bzw. der vom pH-Wert abhängige kationische Polyelektrolyt somit sozusagen die Funktion eines pH-Schalters (d. h. vom pH-Wert abhängig) in bezug auf das Anlösen, Auflösen, Solubilisieren bzw. Ablösen einer einen derartigen Polyelektrolyten und/oder ein derartiges Tensid umfassenden Hüllschicht. Derartige Substanzen ergeben beispielsweise in einer schwach sauren Flüssigwaschmittelformulierung eine stabile Kapselhülle, die eine gute Schutzwirkung für die Peroxycarbonsäure besitzt. Bei Anstieg des pH-Wertes in der Waschflotte lösen sich diese Substanzen besser auf.

Erfindungsgemäß kann eine Vielzahl von Hüllschichten auf die mindestens eine organische Peroxycarbonsäure, insbesondere Imidoperoxycarbonsäure, organische Peroxycarbonsäure, insbesondere Imidoperoxycarbonsäure, aufgebracht werden. Dabei kann die Anzahl an Hüllschichten insbesondere mindestens zwei und bis zu zehn oder mehr Hüllschichten betragen. In diesem Zusammenhang hat die Anmelderin herausgefunden, daß zum Erreichen einer effektiven Verkapselung bzw. Schutzwirkung in bezug auf die Peroxycarbonsäure einerseits und einer guten Freisetzung der zu verkapselnden Peroxycarbonsäure in der Waschflotte andererseits insbesondere mindestens zwei und vorzugsweise bis zu zehn oder mehr Hüllschichten zu einem optimalen Ergebnis führen. Dabei hat die Anmelderin gefunden, daß -insbesondere-bei- Kapselsystemen mit acht oder mehr Hüllschichten gute Ergebnisse hinsichtlich einer effektiven Verkapselung und einer guten Freisetzung der zu verkapselnden Peroxycarbonsäure erzielt werden können.

Bei dem erfindungsgemäßen Verfahren sollten die in Verfahrensschritt (a), (b) und/oder gegebenenfalls (c) bereitgestellten, mindestens einen Polyelektrolyten bzw. mindestens ein ionisches Tensid enthaltenen Lösungen oder Dispersionen einen pH-Wert von höchstens 6, insbesondere einen pH-Wert von 1 bis 6, vorzugsweise von 2 bis 5, bevorzugt von 3 bis 4, besonders bevorzugt von 3,5 aufweisen, um einen Abbau der bei einem pH-Wert ≤ 3,5 relativ wirkungsvoll zu stabilisierenden, aber bei neutralen oder alkalischen pH-Werten relativ schnell zersetzbaren Peroxycarbonsäure bei der Herstellung der erfindungsgemäßen Kapselsysteme zumindest im wesentlichen zu verhindern oder zu verringern.

Im Rahmen des erfindungsgemäßen Verfahrens kann in Verfahrensschritt (a) und (b) und gegebenenfalls (c) das Aufbringen der Lösung bzw. Dispersion auf die zu stabilisierende Peroxycarbonsäure bzw. auf die vorangehende Hüllschicht in einer Wirbelschichtanlage und/oder durch Dragierkessel, Trommelcoater, Mischer, insbesondere Sprühtrockner, Wurstercoater, vorzugsweise durch Aufsprühen der Lösung bzw. Dispersion in einer Wirbelschichtanlage, erfolgen.

Dabei kann im Rahmen einer technischen Herstellung gemäß einem bevorzugten Verfahren in einer Wirbelschichtanlage abwechselnd mit zwei Düsen gearbeitet werden, mit denen die jeweiligen Lösungen bzw. Dispersionen des jeweiligen Hüllschichtmaterials auf die zu stabilisierende Peroxycarbonsäure bzw. auf die vorangehende Hüllschicht aufgebracht werden, so daß grundsätzlich beliebig viele Hüllschichten aufgebracht werden können. Die erforderliche Prozeßzeit wird dabei durch die zu verdampfende Wassermenge vorgegeben.

Gemäß einer bevorzugten Ausführungsform kann z. B. die erste Schicht mittels eines Sprühtrockners und die zweite, dritte, vierte etc. Schicht z. B. mittels eines Wurstercoaters, Dragierkessels, Trommelcoaters, Mischers, vorzugsweise mittels eines Wurstercoaters aufgebracht werden.

Durch das erfindungsgemäße Verfahren können die einzelnen Schichten somit auf einfache Weise aufgebracht werden, ein aufwendiges Entfernen nichtadsorbierter Substanzen zwischen dem Aufbringen der Hüllschichten ist nicht erforderlich.

Im Rahmen des Aufbringens insbesondere der ersten Hüllschicht können sich gegebenenfalls Agglomerate aus den festen Peroxycarbonsäureteilchen bilden, so daß hierdurch eine Einstellung der gewünschten Teilchengröße möglich ist. In diesem Zusammenhang kann somit auch ein Kapselsystem hergestellt werden, das mehrere Kapselkerne umfaßt.

Mit Hilfe des erfindungsgemäßen Herstellungsverfahrens können Schichtdicken in einem großen Bereich ausgebildet werden, die signifikant dicker sind als eine molekulare Schicht, so daß eine aufeinanderfolgende bzw. alternierende Abfolge aus Grenzschichten, welche die zuvor beschriebenen Komplexe umfassen, und Schichten aus nichtkomplexierten Polyelektrolyten bzw. ionischen Tensiden ausgebildet werden kann. Hieraus resultieren besonders positive Eigenschaften des erfindungsgemäßen Kapselsystems hinsichtlich der Lagerung und Freisetzung der Peroxycarbonsäure.

Falls erforderlich oder anwendungsbezogen gewünscht, können dem Kernmaterial (= Peroxycarbonsäure) und/oder der Kapselhülle noch weitere Zusatz- oder Hilfsstoffe zugegeben sein (z. B. Substanzen, die der Erhöhung der Handhabungssicherheit dienen, wie Borsäure, Stabilisatoren, Modifizierungsmittel, anorganische Salze, Farbstoffe).

So kann im Rahmen der vorliegenden Erfindung mindestens einer Hüllschicht mindestens ein Komplexbildner zugegeben werden, der insbesondere ausgewählt sein kann aus der Gruppe von Chinolin und/oder seinen Salzen, Phosphaten, Alkalimetallpolyphosphonaten, Picolinsäure und Dipicolinsäure, Mono- oder Polyphosphonsäuren, insbesondere - 1-Hydroxyethyliden-1,1-diphosphonsäure (HEDP), Ethylendiamintetraessigsäure (EDTA), Diethylentriaminpenta(methylenphosphonsäure) (DTPMP), Azacycloheptandiphosphonat (AHP) bzw. Nitrilotriessigsäure (NTA), insbesondere zur Komplexierung von Schwermetallionen, so daß Schwermetallionen, die insbesondere Abbau-, Oxidations- bzw. Reduktionsreaktionen und/oder Hydrolysereaktionen in bezug auf die Peroxycarbonsäure, beispielsweise PAP, katalysieren, wirkungsvoll gebunden-werden.

Zusätzlich kann mindestens einer Hüllschicht mindestens ein Weichmacher, insbesondere mindestens ein Weichmacher für wasserlösliche Polymere, vorzugsweise Polyethylenglykol (PEG), Glycerin, Glykol oder Triacetin, zugegeben werden, wodurch die mechanischen Eigenschaften der Kapselhülle bzw. der Hüllschichten und damit die mechanischen Eigenschaften des Kapselsystems beeinflußt werden können.

Weiterhin kann mindestens einer Hüllschicht mindestens ein Abpuderungsmittel zugegeben werden, wodurch die Klebrigkeit des Materials verringert und dadurch dessen Prozeßfähigkeit verbessert werden kann. Dabei sollte das Abpuderungsmittel vorzugsweise nicht alkalisch reagieren. Erfindungsgemäß bevorzugte Abpuderungsmittel sind beispielsweise Sulfatsalze und Kieselsäure, z. B. Sipemat^{®} der Firma Degussa.

Im allgemeinen kann bei dem erfindungsgemäßen Verfahren in Verfahrensschritt (a), (b) und/oder gegebenenfalls (c) ein Aufbereiten, insbesondere ein Trocknen bzw. ein Aufreinigen bzw. ein Klassieren des Kapselsystems, beispielsweise nach Aufbringen der jeweiligen Hüllschicht(en), durch übliche Methoden erfolgen, beispielsweise durch Gefriertrocknung (Lyophilisierung), Verdampfen des Dispersionsmittels, vorzugsweise bei einer Temperatur von etwa 40 °C bis 60 °C in einer Wirbelschichtanlage, Ultrafiltration, Dialyse, Sprühtrocknung unter schonenden Bedingungen oder Sieben.

Das in Verfahrensschritt (d) gegebenenfalls durchgeführte Aufbereiten, -beispielsweise Trocknen und/oder Aufreinigen bzw. Klassieren, kann ebenfalls durch übliche, Methoden - wie oben beschrieben - erfolgen. In diesem Zusammenhang kann auch eine Formgebung in bezug auf das Kapselsystem, beispielsweise durch Verrunden oder dergleichen, erfolgen.

Bei dem erfindungsgemäßen Verfahren können mit mindestens einer organischen Peroxycarbonsäure, insbesondere Imidoperoxycarbonsäure, beladene Kapselsysteme mit einer mittleren Größe (Kugeldurchmesser, Granulometrie) von 20 µm bis 4.000 µm, vorzugsweise 50 µm bis 3.000 µm, bevorzugt 100 µm bis 2.000 µm, erhalten werden, so daß eine gezielte Anpassung der Größe des Kapselsystems entsprechend seiner jeweiligen späteren Verwendung möglich ist.

Gegebenenfalls kann - falls dies anwendungsbezogen erforderlich oder gewünscht sein sollte - eine Auftrennung des erfindungsgemäßen Kapselsystems entsprechend seiner Größe bzw. Granulometrie erfolgen, beispielsweise mittels Sieben.

Bei dem erfindungsgemäßen Verfahren kann der Anteil der jeweiligen Hüllschicht der Kapselhülle 1 bis 15 Gew.-%, vorzugsweise 1,5 bis 10 Gew.-%, bevorzugt 2 bis 10 Gew.-%, bezogen auf das Kapselsystem, betragen. Dabei kann der Anteil der gesamten, die jeweiligen Hüllschichten umfassenden Kapselhülle bis zu 70 Gew.-%, bezogen auf das Kapselsystem, betragen. Das Molekulargewicht des Polyelektrolyten in der jeweiligen Hüllschicht sollte ≥ 1.000, vorzugsweise ≥ 10.000, bevorzugt ≥ 15.000, sein. Der Gehalt an organischer Peroxycarbonsäure, insbesondere Imidoperoxycarbonsäure, vorzugsweise PAP, kann ≥ 30 Gew.-%, vorzugsweise ≥ 40 Gew.-%, bevorzugt ≥ 50 Gew.-%, bezogen auf das Kapselsystem, betragen. Je nach Anwendung sollte dabei eine Anpassung des Gehalts an Peroxycarbonsäure beispielsweise vor dem Hintergrund der Erhöhung der Handhabungssicherheit des erfindungsgemäßen Kapselsystems vorgenommen werden. So kann gegebenenfalls aus Gründen der Produktsicherheit ein allzu hoher Gehalt an Peroxycarbonsäuren nicht wünschenswert bzw. praktikabel sein. Für diese Fälle sollte der Gehalt an Peroxycarbonsäure beispielsweise 50 Gew.-%, bezogen auf das Kapselsystem, nicht überschreitet.

Gemäß einer erfindungsgemäß besonders bevorzugten Ausführungsform, bei dem die Kapselhülle des Kapselsystems vier Hüllschichten umfaßt, beträgt der Anteil der ersten, mindestens ein kationisches Tensid bzw. Polymer umfassenden Hüllschicht sowie der dritten, mindestens ein kationisches Polymer bzw. kationisches Tensid umfassenden Hüllschicht jeweils 2 bis 5 Gew.-% und der Anteil der zweiten sowie vierten, jeweils mindestens einen anionischen Polyelektrolyten bzw. mindestens ein anionisches Tensid umfassenden Hüllschichten beträgt jeweils 5 bis 10 Gew.-%, jeweils bezogen auf das Kapselsystem.

Die erfindungsgemäß bevorzugten Anteile der jeweiligen Hüllschichten werden vor dem Hintergrund ausgewählt, eine effektive Schutzfunktion sowie eine gute Freisetzung hinsichtlich der zu verkapselnden Peroxycarbonsäure in der Waschflotte zu ermöglichen. Herbei werden erfindungsgemäß - wie bereits angeführt - besonders bevorzugt Hüllsysteme aufgebracht, deren Hüllschichtdicken deutlich größer sind als die einer Schicht mit molekularer Dicke, so daß es zur Ausbildung einer komplexierten Grenzschicht zwischen den einzelnen Hüllschichten sowie Schichten mit nichtkomplexierten, insbesondere in der Wasch- und Reinigungsflotte wasserlöslichen Polyelektrolyten und/oder ionischen Tensiden kommen kann.

Das nach dem erfindungsgemäßen Verfahren hergestellte Kapselsystem weist einen sogenannten "Controlled-released-Effekt" ("Effekt der kontrollierten Freisetzung") auf. Unter einem "Controlled-released-Effekt" ist insbesondere eine geringfügige, vorzugsweise zwischen 1 und 15 Minuten betragende, Verzögerung der Auflösung der Kapseln bei ihrer Anwendung, zum Beispiel in einer Waschflotte, bzw. eine Verzögerung der Freisetzung der Peroxycarbonsäure aus dem erfindungsgemäßen Kapselsystem zu verstehen.

Im Rahmen des erfindungsgemäßen Verfahrens kann abschließend zusätzlich das Aufbringen einer weiteren Hülle bzw. Hüllschicht ("Coating") auf das erfindungsgemäße Kapselsystem vorgesehen sein, wodurch ein- zusätzlicher stabilisierender Effekt in bezug auf die zu stabilisierende bzw. zu verkapselnde organische Peroxycarbonsäure bewirkt wird. Beispielsweise kann in dieser zusätzlichen Hülle ein Komplexbildner inkorporiert sein, der Schwermetallionen komplexieren kann, so daß eine schwermetallkatalysierte Zersetzung der Peroxycarbonsäure zumindest weitgehend unterbunden werden kann. Das Aufbringen einer zusätzlichen Hülle kann weiterhin dazu dienen, die Auflösegeschwindigkeit weiter zu modifizieren und diese in gewünschter Weise einzustellen; hierdurch kann ein zusätzlicher "Controlled-release-Effekt" bezüglich der in dem erfindungsgemäßen Kapselsystem enthaltenen Peroxycarbonsäure erzielt werden. Das Aufbringen der zusätzlichen Hülle ("Coaten") kann in einer dem Fachmann an sich bekannten Weise durchgeführt werden, z. B. über Wirbelschichtverfahren oder durch Adsorption des zusätzlichen Hüllmaterials ("Coatingmaterials") auf das Kapselsystem aus einer Lösung, Aufsprühen einer Lösung oder Schmelze des Hüllmaterials auf das Kapselsystem und anschließende Verdampfung des Lösungsmittels, vorzugsweise Wasser, oder mittels Umhüllung ("Coating") im Mischer, Kessel, Dabei können erfindungsgemäß dem Fachmann an sich bekannte Materialien als zusätzliche Hüllsubstanzen eingesetzt werden, wie beispielsweise anorganische Verbindungen, z. B. Salze und anorganische Oxide, insbesondere Sulfate oder Phosphate, oder aber hochmolekulare Verbindungen, wie organische Polymere, z. B. Celluloseether, Polyvinylalkohol (PVAI), Polyvinylpyrrolidon (PVP).

Darüber hinaus kann bei dem erfindungsgemäßen Verfahren beispielsweise auf die organische Peroxycarbonsäure, vorzugsweise vor Aufbringen der Hüllschichten bzw. Kapselhülle, eine Substanz aufgebracht werden, welche bei einer Temperatur unterhalb von 80 °C, insbesondere unterhalb von 70 °C, insbesondere mit sich selbst endotherme Reaktionen, beispielsweise Kristallwasserabspaltungsreaktionen oder Zersetzungsreaktionen, eingehen kann. Diese Substanz kann erfindungsgemäß auch mit der Peroxycarbonsäure vermengt, insbesondere vermischt sein. Eine derartige Substanz ist beispielsweise Borsäure. Im Rahmen der vorliegenden Erfindung kann diese Substanz, vorzugsweise vor Aufbringen der Hüllschichten bzw. Kapselhülle, direkt auf die Peroxycarbonsäure aufgebracht werden, wobei beispielsweise dieselben Verfahrensschritte wie zur Bildung der weiteren Hülle ("Coating") eingesetzt werden können. Die zugegebene Substanz führt zu einer Erhöhung der Handhabungssicherheit des erfindungsgemäßen Kapselsystems, da sie eine gegebenenfalls auftretende Wärmetönung abfangen bzw. kompensieren kann. Unter einer Wärmetönung kann eine in dem Kapselsystem lokal auftretende Temperaturerhöhung, die durch eine lokal stattfindende bzw. beginnende exotherme Zersetzung der Peroxycarbonsäure hervorgerufen werden kann, aber auch eine in einem Gebinde bzw. in der Dispersion selbst- beispielsweise bei Lagerung- auftretende Temperaturerhöhung verstanden werden. Die zugegebene Substanz, beispielsweise Borsäure, kann auch in die Hüllschichten bzw. Kapselhülle eingebracht werden. Erfindungsgemäß bevorzugt ist jedoch ein Aufbringen auf die Peroxycarbonsäure bzw. ein Vermengen bzw. Vermischen mit der Peroxycarbonsäure, da dies zu einer höheren Effektivität hinsichtlich der Handhabungssicherheit führt.

Das nach dem erfindungsgemäßen Verfahren in Verfahrensschritt (b), (c) und/oder gegebenenfalls (d) erhaltene Kapselsystem kann beispielsweise zusammen mit weiteren Inhaltsstoffen zu einem Wasch- oder Reinigungsmittel, insbesondere einem flüssigen Wasch- oder Reinigungsmittel, formuliert werden. Dabei sollte das Wasch- oder Reinigungsmittel zumindest im wesentlichen keine Halogenidionen, insbesondere keine Chloridionen aufweisen bzw. die Menge an Halogenidionen, insbesondere Chloridionen, höchstens 500 ppm, vorzugsweise höchstens 100 ppm, besonders bevorzugt höchstens 30 ppm betragen. Der pH-Wert sollte höchstens 7 betragen, insbesondere zwischen 3,5 und 7, vorzugsweise zwischen 4,0 und 6,5, besonders bevorzugt zwischen 4,5 und 6, liegen. Weiterhin kann das Wasch- oder Reinigungsmittel mindestens einen Komplexbildner enthalten; dieser kann beispielsweise ausgewählt sein aus der Gruppe von Chinolin und/oder seinen Salzen, Alkalimetallpolyphosphonaten, Picolinsäure und Dipicolinsäure, Mono- oder Polyphosphonsäuren, insbesondere 1-Hydroxyethyliden-1,1-diphosphonsäure (HEDP), Ethylendiamintetraessigsäure (EDTA), Diethylentriaminpenta(methylenphosphonsäure) (DTPMP), Azacycloheptandiphosphonat (AHP), Nitrilotriessigsäure (NTA), Citrat und/oder kurzkettige dicarbonsäuren; diese Komplexbildner werden im -Rahmen des erfindungsgemäßen Verfahrens insbesondere zur Komplexierung von Schwermetallionen eingesetzt. Weiterhin kann das Wasch- oder Reinigungsmittel gegebenenfalls mindestens ein wassermischbares Lösemittel mit einem für die organische Peroxycarbonsäure, insbesondere Imidoperoxycarbonsäure, geringen Lösungsvermögen aufweisen (z. B. in Mengen von vorzugsweise mehr als 20 Gew.-%, besonders bevorzugt mehr als 30 Gew.-%, bezogen auf das Wasch- oder Reinigungsmittel) oder aber das wassermischbare Lösemittel ist das Dispersionsmittel der Dispersion. Beispielsweise kann das Lösemittel Glycerin sein. Das erfindungsgemäß bevorzugte Lösemittel ist jedoch Wasser. Zudem kann dem Wasch- oder Reinigungsmittel gegebenenfalls mindestens eine Katalase zugesetzt werden. Zu weiteren Einzelheiten in bezug auf das Wasch- oder Reinigungsmittel kann auf nachfolgende Ausführungen verwiesen werden.

Das erfindungsgemäße Verfahren ist gleichermaßen ein Verfahren zur Stabilisierung von Peroxycarbonsäuren, insbesondere Imidoperoxycarbonsäuren, vorzugsweise PAP, bzw. ein Verfahren zur Erhöhung der Lagerfähigkeit von Peroxycarbonsäuren, insbesondere Imidoperoxycarbonsäuren, vorzugsweise PAP.

Gemäß einer typischen Ausführungsform kann das erfindungsgemäße Verfahren wie folgt durchgeführt werden: Zur Verkapselung bzw. zum Schutz der Peroxycarbonsäure, insbesondere Imidoperoxycarbonsäure (z. B. PAP), die in form fester Teilchen, insbesondere in kristalliner Form, vorliegt, wird eine Kapselhülle im Rahmen eines Mehrschichtverfahrens auf die Peroxycarbonsäure aufgebracht. Dazu wird als erste Schicht auf PAP ein Polyelektrolyt oder ein Tensid mit einer positiven Nettoladung aufgebracht. Da PAP-Kristalle auch bei schwach sauren pH-Werten eine kleine negative Oberflächenladung besitzen, ist es vorteilhaft, für diese erste Schicht ein kationisches Material zu verwenden. Als Kationtenside können übliche kationische Tenside, z. B. quaternäre Ammoniumsalze, deren Ammoniumrest der allgemeinen Formel R¹R²R³N⁺ entspricht, wobei die Reste R¹, R² und R³, identisch oder verschieden, ein Wasserstoffatom oder einen linearen oder verzweigten Alkyl-, Alkylen- oder Alkinylrest mit 1 bis 40 Kohlenstoffatomen, insbesondere 1 bis 25 Kohlenstoffatomen, darstellen, wie Alkyldimethylammoniumtenside, Esterquats und ferner N-Alkylpyridiniumsalze eingesetzt werden. Es hat sich als vorteilhaft erwiesen, wenn die Gegenionen der Tenside keine Halogenidionen sind. Geeignet sind beispielsweise Methylsulfat-, Sulfat-, Phosphat-, Tosylat- oder Cumolsulfonatanionen. Alternativ kann als Material für die erste Schicht ein kationisches Polymer verwendet werden. Geeignet sind quatemisierte Amin-, Imin- oder Imidazolgruppen tragende Polymere, beispielsweise Polydiallyldimethylammoniumpolymere. Ferner eignen sich alle, beispielsweise von kosmetischen Anwendungen bekannte Polymere der allgemeinen Bezeichnung "Polyquaternium^{®}", wobei besonders solche geeignet sind, deren Gegenionen keine Halogenidionen, insbesondere Chiaridionen sind. Als zweite Schicht wird ein Polymer entgegengesetzter Ladung aufgebracht. Daals erste Schicht ein kationisches Material zum Einsatz kommt, folgt nun ein anionisches Material. Besonders bevorzugt sind hier synthetische Polymere, wie Polycarbonsäuren und deren partielle Salze. Geeignet sind beispielsweise Polyacrylsäure, Polymaleinsäure und deren Copolymere. Ferner eignen sich polymere Sulfonsäuren, beispielsweise Polystyrolsulfonsäure und deren partielle Salze. Auch polymeranalog sulfonierte Polymere sind möglich. Weiterhin können auch natürliche anionische Polymere eingesetzt werden, wie beispielsweise Alginsäure, Xanthan etc. Darüber hinaus sind auch derivatisierte natürliche Polymere, wie Carboxymethylcellulose, möglich. Optional kann nun eine dritte, vierte etc. Schicht aufgebracht werden, die jeweils aus zur vorangegangenen Schicht gegensinnig geladenem Material besteht. Für die zweite, dritte, vierte etc. Schicht sind Polymere gegenüber den Tensiden bevorzugt, Tenside sowie Mischungen aus Polymeren mit gleichartig geladenen Tensiden können aber ohne weiteres verwendet werden. Im Rahmen der vorliegenden Erfindung werden die Materialien der Hüllschichten z. B. abwechselnd in einer Wirbelschichtanlage als saure, bevorzugt mit einem pH-Wert von etwa 3,5, wäßrige Lösung aufgesprüht. Es ist zweckmäßig, in einer Wirbelschichtanlage zu arbeiten, in welcher das Beschichtungsmaterial über mindestens zwei Düsen, die aus unterschiedlichen Vorratstanks gespeist werden, ein- bzw. aufgesprüht werden kann. Durch alternierendes Sprühen aus den beiden Düsen oder durch räumlichen Transport des Beschichtungsgutes (Peroxycarbonsäureteilchen, unfertiges Kapselsystem) von einem Bereich, der mit einer Lösung bzw. Dispersion besprüht wird, zu einem anderen Bereich, der mit der anderen Lösung bzw. Dispersion besprüht wird, kann so das Aufbringen der Hüllschichten bzw. der mehrschichtigen Kapselhülle ohne großen Reinigungs- oder Umbauaufwand an der Apparatur hergestellt werden. Ferner eignen sich, insbesondere zum Aufbringen der ersten Schicht, Sprühtrockner und für die weiteren Schichten Wurstercoater, aber auch andere Apparate zur Beschichtung makroskopischer Teilchen, z.B. Dragierkessel, Trommelcoater, Mischer etc. Beim Aufsprühen der ersten Schicht können sich optional Agglomerate einer gewünschten Teilchengröße bilden. Die Teilchengröße liegt im Bereich von 50 bis 3.000 µm, bevorzugt von 100 bis 200 µm. Beim Aufsprühen jeder weiteren Schicht bildet sich an der Grenzschicht zur vorausgegangenen Schicht ein Grenzschicht-Komplex (z. B. ein Polyanion/Polykation-Komplex etc.). Dieser ist in Abhängigkeit von der Stöchiometrie, wasserlöslich, mäßig wasserlöslich oder - insbesondere im Falle von Ladungsneutralisation - unlöslich; diesbezüglich kann auf obige Ausführungen verwiesen werden.

Dabei wurde überraschenderweise gefunden, daß diese in einem Mehrschrittverfahren hergestellten Schichten einen besonders guten Schutz für PAP in einer wasserbasierten flüssigen Wasch- oder Reinigungsmittelformulierung, insbesondere in einer wasserbasierten flüssigen Wasch- oder Reinigungsmittelformulierung bieten. Besonders vorteilhafte Kapselsysteme nach der vorliegenden Erfindung können erhalten werden, indem eine Klasse von Hüllschichten - bevorzugt die kationischen Hüllschichten - nicht aus permanent kationischen Substanzen, sondern aus von dem pH-Wert abhängigen kationischen Substanzen besteht bzw. diese aufweist. Als pH-Wertabhängige kationische Tenside, die bei sauren pH-Werten protoniert vorliegen, bei neutralen pH-Werten hingegen neutral, eignen sich aminoxid- oder phosphinoxidbasierte Tenside, ferner pyridiniumbasierte Tenside. Als von dem pH-Wert abhängige kationische Polymere eignen sich solche, die Aminogruppen, Iminogruppen, Aminoxidgruppen, Phosphinoxidgruppen oder Pyridin-N-oxidgruppen aufweisen. Ein Beispiel ist Polyvinylpyridin-N-oxid. Diese Substanzen ergeben in einer schwach sauren Flüssigwaschmittelformulierung eine stabile Kapselhülle, die eine gute Schutzwirkung für das Bleichmittel (z. B. PAP) besitzt. Bei Neutralisierung des pH-Wertes in der Waschflotte lösen sie sich besser auf. Die erfindungsgemäßen Beschichtungen können weitere Substanzen enthalten. Diese können dazu dienen, Schwermetallionen, die den Abbau des Bleichmittels fördern, unschädlich zu machen. Besonders geeignet sind Komplexbildner, beispielsweise Nitrat, EDTA, Phosphate und besonders bevorzugt Phosphonate, wie HDEP. Weitere Zusatzstoffe können beispielsweise dazu- dienen, die mechanischen Eigenschaften der Schichten positiv zu beeinflussen. Beispielsweise können hierzu Weichmacher verwendet werden. Geeignet sind übliche Weichmacher für wasserlösliche Polymere, beispielsweise Polyethylenglykol (PEG), Glycerin, Glykole oder Triacetin etc. Gemäß dieser typischen Ausführungsform der vorliegenden Erfindung wird somit ein mehrschichtiges Kapselsystem, welches ein Bleichmittel, bevorzugt PAP, als Kernmaterial mit einer mehrschichtigen, mindestens zweischichtigen Kapselhülle aus jeweils gegensinnig geladenen ionischen Tensiden und/oder ionischen Polymeren enthält.

Gemäß einem zweiten Aspekt ist ein weiterer Gegenstand der vorliegenden Erfindung das nach dem erfindungsgemäßen Verfahren erhältliche Kapselsystem.

Die zu verkapselnde organische Peroxycarbonsäure ist insbesondere ausgewählt aus Mono- und Diperoxycarbonsäuren, insbesondere Dodekandiperoxysäure und vorzugsweise Imidoperoxysäuren, besonders bevorzugt 6-Phthalimidoperoxycapronsäure (6-Phthalimidoperoxyhexansäure, PAP). Dabei sollte die Peroxycarbonsäure bei Atmosphärendruck einen Schmelzpunkt oberhalb von 25 °C besitzen, insbesondere oberhalb von 35 °C, vorzugsweise oberhalb von 45 °C, bevorzugt oberhalb von 50 °C, besonders bevorzugt oberhalb von 100 °C.

Erfindungsgemäß ist die mehrschichtige, mindestens zwei Hüllschichten umfassende Kapselhülle, welche die zu verkapselnde Peroxycarbonsäure umgibt, derart, daß die zu verkapselnde Peroxycarbonsäure vollständig von der Kapselhülle umschlossen ist, so daß die Peroxycarbonsäure zumindest im wesentlichen nicht in direktem Kontakt in bezug auf das Umgebungsmilieu, insbesondere Dispersionsmittel, steht, d. h. die zu verkapselnde Peroxycarbonsäure liegt in dem erfindungsgemäßen Kapselsystem als Kernmaterial vor, welches von der mehrschichtigen, mindestens zwei Hüllschichten umfassenden Kapselhülle umgeben ist.

Für weitere Einzelheiten in bezug auf das erfindungsgemäße Kapselsystem kann auf obige Ausführungen zu dem erfindungsgemäßen Verfahren verwiesen werden, welche für das erfindungsgemäße Kapselsystem entsprechend gelten.

Bei dem erfindungsgemäßen Kapselsystem beträgt der Anteil der jeweiligen Hüllschicht der Kapselhülle 1 bis 15 Gew.-%, vorzugsweise 1,5 bis 10 Gew.-%, bevorzugt 2 bis 10 Gew.-%, bezogen auf das Kapselsystem. Dabei kann der Anteil der gesamten, die jeweiligen Hüllschichten umfassenden Kapselhülle bis zu 70 Gew.-%, bezogen auf das Kapselsystem, betragen. Das Molekulargewicht des Polyelektrolyten in den jeweiligen Hüllschichten sollte ≥ 1.000, vorzugsweise ≥ 10.000, bevorzugt ≥ 15.000 betragen. Der Gehalt an organischer Peroxycarbonsäure, insbesondere Imidoperoxycarbonsäure, beispielsweise PAP, beträgt ≥ 30 Gew.-%, vorzugsweise ≥ 40 Gew.-%, bevorzugt ≥ 50 Gew.-%, bezogen auf das Kapselsystem.

Im Rahmen der vorliegenden Erfindung kann die Freisetzung der zu verkapselnden Peroxycarbonsäure, insbesondere Imidoperoxycarbonsäure, beispielsweise PAP, aus dem Kapselsystem bei der Anwendung insbesondere in einer Waschflotte erfolgen. Dabei kann die Freisetzung insbesondere durch An- bzw. Auflösen, Dispergieren bzw. Solubilisieren der Hüllschichten, beispielsweise durch Erhöhung des pH-Wertes bzw. Tensidreaktivierung, insbesondere Tensidwirkung, und Einwirkung der Waschmechanik in der Waschflotte, erfolgen. Wenn es sich z. B. um ein Wasch- oder Reinigungsmittelkonzentrat handelt, welches das erfindungsgemäße Kapselsystem sowie Tenside in inaktivierter Form (z. B. durch Aussalzen beispielsweise mit Natriumsulfat bzw. in Form von Flüssigkristallen) enthält, so werden bei Verdünnung dieses Konzentrats im Rahmen der Anwendung in der Waschflotte die Tenside aus ihrer inaktivierten bzw. ausgesalzenen Form in ihre aktive Form überführt, so daß die auf diese Weise aktivierten Tenside die Hüllschichten bzw. Kapselhülle an- bzw. auflösen, dispergieren bzw. solubilisieren können. Beim Verdünnen in der Waschflotte tritt gleichzeitig ein pH-Wert-Sprung ein, so daß die Löslichkeit der Peroxycarbonsäure deutlich zunimmt. Die Freisetzung der Peroxycarbonsäure, insbesondere Imidoperoxycarbonsäure (PAP), aus dem Kapselsystem kann in der Waschflotte auch durch osmotische Prozesse und/oder Diffusionsvorgänge erfolgen. So können beispielsweise Wassermoleküle entlang des Konzentrationsgradienten durch die semipermeable, d. h. für Wasser permeable und für Diperoxycarbonsäure impermeable Kapselhülle in den Kernbereich des Kapselsystems diffundieren und dort bei einem entsprechenden pH-Wert der Waschflotte, insbesondere bei einem pH-Wert ≥ 7, zu einem An- bzw. Auflösen der Peroxycarbonsäure führen. Ohne sich auf eine Theorie festlegen zu wollen, kann hierdurch ein hoher osmotischer Druck in der Kapsel generiert werden, der sozusagen zu einem Aufplatzen der Kapselhülle mit einhergehender Freisetzung der Peroxycarbonsäure in der Waschflotte führen kann. Ohne sich auf eine Theorie festlegen zu wollen, kann ein An- bzw. Auflösen der Kapselhülle bzw. der Hüllschichten durch eine Erhöhung des pH-Wertes in der Waschflotte dadurch erfolgen, daß - wie bereits angeführt - kationische Polyelektrolyte bzw. kationische Tenside, deren elektrische Ladung von dem pH-Wert abhängt, neutralisiert werden und somit die Kapselhülle leichter an- bzw. aufgelöst werden kann. Schließlich sind auch mechanische Vorgänge von Bedeutung, z. B. mechanische Zerstörung des Kapselsystem durch die in der Waschflotte befindlichen Wäschestücke bzw. durch Kontakt mit der Waschtrommel. Insbesondere ist auch eine Kombination der einzelnen vorgenannten Prozesse in bezug auf die Freisetzung der Peroxycarbonsäure aus den erfindungsgemäßen Kapselsystemen möglich.

Das erfindungsgemäße Kapselsystem weist zahlreiche Verwendungsmöglichkeiten auf. So kann das erfindungsgemäße Kapselsystem - gemäß einem weiteren Aspekt der vorliegenden Erfindung - in Wasch- und Reinigungsmitteln, insbesondere flüssigen Wasch- und Reinigungsmitteln, Zahnpflegemitteln, Haarfärbemitteln und für Entfärbungs- bzw. Bleichmittelzusammensetzungen für technische Anwendungen eingesetzt werden.

In diesem Zusammenhang kann das erfindungsgemäße Kapselsystem auch als "Delivery-System" zur kontrollierten Freisetzung von Peroxycarbonsäuren eingesetzt werden, wobei die Freisetzung der Peroxycarbonsäure insbesondere durch die Zusammensetzung bzw. durch die Anzahl der die Peroxycarbonsäure zumindest im wesentlichen umgebenden Hüllschichten kontrolliert werden kann. Unter Zusammensetzung wird hierbei erfindungsgemäß insbesondere die Art und/oder Menge des entsprechenden Polyelektrolyten bzw. des entsprechenden ionischen Tensids in der jeweiligen Hüllschicht verstanden. Dabei kann eine Steuerung der Freisetzung der Peroxycarbonsäure insbesondere über die Wasserlöslichkeit der als solche bzw. der Wasserlöslichkeit in Abhängigkeit von dem pH-Wert des Dispersionsmittels in bezug auf die jeweilige Hüllschicht erfolgen. Eine weitere Modifikationsmöglichkeit stellt die Aufbringung einer zusätzlichen Hülle ("Coating") auf das erfindungsgemäße Kapselsystem dar.

Das erfindungsgemäße Kapselsystem kann insbesondere als "Delivery-System" verwendet werden, bei dem die Peroxycarbonsäuren über einen großen Zeitraum durch eine verlängerte bzw. verzögerte Freisetzung abgegeben werden ("Sustained-release-Effekt").

Ein weiterer Gegenstand der vorliegenden Erfindung - gemäß einem weiteren Aspekt der vorliegenden Erfindung - sind Wasch- und Reinigungsmittel, insbesondere flüssige Wasch- und Reinigungsmittel, Zahnpflegemittel, Haarpflegemittel, Färbungs- bzw. Bleichmittelzusammensetzung für technische Anwendungen, welche das erfindungsgemäße, mit mindestens einer organischen Peroxycarbonsäure, insbesondere Imidoperoxycarbonsäure, beladene mehrschichtige Kapselsystem enthalten.

Die erfindungsgemäßen Wasch- und Reinigungsmittel, welche das erfindungsgemäße Kapselsystem enthalten, sind sowohl im Haushalts- als auch im Industriebereich einsetzbar. Insbesondere handelt es sich bei den erfindungsgemäßen Wasch- und Reinigungsmitteln um flüssige Wasch- und Reinigungsmittel, welche das erfindungsgemäße Kapselsystem enthalten.

Die erfindungsgemäßen Wasch- und Reinigungsmittel können zur Reinigung harter Oberflächen und/oder weicher, insbesondere textiler Oberflächen verwendet werden. Die erfindungsgemäßen Wasch- und Reinigungsmittel können insbesondere als Geschirrspülmittel, Allzweckreiniger, Badreiniger, Fußbodenreiniger, Autoreiniger, Glasreiniger, Möbelpflegemittel bzw. -reiniger, Fassadenreiniger, Waschmittel, zum Beispiel verwendet werden, besonders bevorzugt als Waschmittel. Weiterhin sind die erfindungsgemäßen Wasch- und Reinigungsmittel vorzugsweise zur Reinigung von Fasern, Textilien, Teppichen, zum Beispiel, geeignet.

Die erfindungsgemäßen Wasch- und Reinigungsmittel enthalten neben dem erfindungsgemäßen Kapselsystem an sich übliche Inhaltsstoffe bzw. Bestandteile (z.B. -Tenside, Duftstoffe, Farbstoffe, Enzyme, Enzymstabilisatoren, Geruchsstoffe bzw. Builder, pH-Wert-Einstellmittel, andere Bleichmittel, Bleichaktivatoren, Silberschutzmittel, schmutzabweisende Substanzen, optische Aufheller, Vergrauungsinhibitoren, Desintegrationshilfsmittel, Verdickungsmittel, Entschäumer bzw. Schauminhibitoren, Komplexbildner für Schwermetalle, schmutzabweisende Substanzen bzw. Soil-Repellents, Farbübertragungsinhibitoren, Lösungsmittel, optische Aufheller und/oder weitere übliche Inhaltsstoffe), wobei im Rahmen der vorliegenden Erfindung auf die Kompatibilität der einzelnen Inhaltsstoffe bzw. Bestandteile sowohl untereinander als auch im Hinblick auf das erfindungsgemäße Kapselsystem bzw. die darin verkapselten Peroxycarbonsäuren geachtet werden sollte, was durch gezielte Auswahl der Inhaltsstoffe bzw. Bestandteile und/oder ihrer jeweiligen Mengenverhältnisse realisiert wird. Auf diese Weise kann eine unerwünschte Wechselwirkung dieser Inhaltsstoffe bzw. Bestandteile mit dem erfindungsgemäßen Kapselsystem bzw. den darin eingelagerten Peroxycarbonsäuren vermieden werden. Wie im folgenden noch näher ausgeführt, kann durch die gezielte Auswahl bestimmter Inhaltsstoffe bzw. Bestandteile und/oder deren Mengenverhältnisse ein stabilisierender Effekt in bezug auf das erfindungsgemäße Kapselsystem bzw. die darin verkapselten Peroxycarbonsäuren bewirkt werden.

Ein erfindungsgemäßes Wasch- oder Reinigungsmittel, insbesondere flüssiges Wasch- oder Reinigungsmittels, umfaßt beispielsweise die folgenden Inhaltsstoffe:
(i) mit mindestens einer organischen Peroxycarbonsäure, insbesondere Imidoperoxycarbonsäure, beladenes Kapselsystem gemäß der vorliegenden Erfindung, vorzugsweise in Mengen von 0,1 bis 20 Gew.-%; und/oder
(ii) Tenside, insbesondere kationische und/oder anionische Tenside, vorzugsweise in Mengen von 5 bis 30 Gew.-%, und/oder nichtionische Tenside, vorzugsweise in Mengen von 0 bis 30 Gew.-%; und/oder
(iii) gegebenenfalls Elektrolyte, insbesondere anorganische und/oder organische Salze, vorzugsweise Natriumsulfat, vorzugsweise in Mengen von 0 bis 30 Gew.-%; und/oder
(iv) gegebenenfalls Komplexbildner, insbesondere ausgewählt aus der Gruppe von Chinolin und/oder seinen Salzen, Alkalimetallpolyphosphonaten, Picolinsäure und Dipicolinsäure, Mono- oder Polyphosphonsäuren, insbesondere 1-Hydroxyethyliden-1,1-diphosphonsäure (HEDP), Ethylendiamintetraessigsäure (EDTA), Diethylentriaminpenta(methylenphosphonsäure) (DTPMP), Azacycloheptandiphosphonat (AHP), Nitrilotriessigsäure (NTA), Citrat und/oder kurzkettige Dicarbonsäuren, vorzugsweise in Mengen von 0 bis 5 Gew.-%; und/oder
(v) gegebenenfalls Enzyme, wie Proteasen, Amylasen, Cellulasen und/oder Lipasen, gegebenenfalls zusammen mit Enzymstabilisatoren, vorzugsweise in Mengen von 0 bis 10 Gew.-%; und/oder
(vi) gegebenenfalls Builder, insbesondere Fettsäuren, vorzugsweise gesättigte und/oder verzweigte Fettsäuren, insbesondere mit einem Schmelzpunkt unterhalb von 30 °C, und/oder Citronensäure und/oder Citrat, vorzugsweise in Mengen von 0 bis 15 Gew.-%; und/oder
(vii) gegebenenfalls Duftstoffe, vorzugsweise in Mengen von 0 bis 5 Gew.-%; und/oder
(viii) gegebenenfalls Hilfsstoffe, wie Entschäumer, pH-Regulatoren, Rheologiemodifikatoren (Verdicker), Lösungsmittel, Farbstoffe; und/oder
(ix) gegebenenfalls weitere übliche Inhaltsstoffe, wie Aufheller; und/oder
(x) Wasser;
wobei sämtliche Gewichtsangaben auf das Wasch- bzw. Reinigungsmittel bezogen sind.

Bei den erfindungsgemäßen Wasch- bzw. Reinigungsmitteln, insbesondere flüssigen Wasch- und Reinigungsmitteln, können die Tenside in der Wasch- und Reinigungsmittelformulierung inaktiviert sein, insbesondere durch Aussalzen, d. h. Induzierung einer Phasentrennung in eine tensidarme, kontinuierliche Phase und eine vorzugsweise lamellare, im allgemeinen hochviskose, kristalline oder flüssigkristalline tensidreiche Phase, vorzugsweise durch Einbringen einer Sulfatverbindung, besonders bevorzugt Natriumsulfat, in die Wasch- oder Reinigungsmittelformulierung. Dabei wird in der Wasch- oder Reinigungsmittelformulierung insbesondere ein Auf- und/oder Anlösen des Kapselsystems bzw. der organischen Peroxycarbonsäure, insbesondere Imidoperoxycarbonsäure, zumindest weitgehend verhindert bzw. unterbunden. Erfindungsgemäß wird unter dem Begriff "kontinuierliche Phase" das Dispersionsmittel mit den darin gelösten Bestandteilen oder Inhaltstoffen (z. B. Salze, Tenside) verstanden. Erfindungsgemäß bevorzugt ist das Dispersionsmittel Wasser.

In diesem Zusammenhang konnte die Anmelderin zeigen, daß organische Peroxycarbonsäuren, insbesondere PAP, in Gegenwart von aktiven Tensiden (d. h. sich in freier und/oder micellarer Form in der Wasch- oder Reinigungsmittelformulierung befindlichen Tensiden) schnell zersetzt werden, da die Peroxycarbonsäuren durch die Tenside verstärkt gelöst werden und in diesem gelösten Zustand äußerst instabil sind. Dabei führen insbesondere nichtionische Tenside (Niotenside), beispielsweise auf Basis von Alkylpolyglykolether, zu einer beschleunigten Zersetzung der Peroxycarbonsäuren. Durch die Zugabe von Sulfat werden die Tenside zumindest teilweise inaktiviert, was insbesondere durch Aussalzen geschieht, wobei die Tenside aus der insbesondere micellaren, aktiven Form in eine vorzugsweise lamellare, kristalline oder flüssigkristalline Form (Kristall- oder Flüssigkristallbildung) überführt werden. Diese Flüssigkristalle selbst, die beispielsweise durch Zentrifugation abgetrennt werden können, sollen eine hohe Viskosität aufweisen. Ferner sollte der Gehalt an freien Tensiden in den erfindungsgemäßen Wasch- und Reinigungsmittelformulierungen bzw. in der kontinuierlichen Phase der erfindungsgemäßen Wasch- und Reinigungsmittelformulierungen allenfalls 1 % betragen.

Die Sulfatkonzentration in dem erfindungsgemäßen Wasch- oder Reinigungsmittel sollte derart gewählt sein, daß bei der Anwendung des Wasch- oder Reinigungsmittels in der Waschflotte die Tenside wieder in aktiver Form vorliegen, was beispielsweise durch einen Verdünnungseffekt beim Eintragen des Wasch- oder Reinigungsmittels in die Waschflotte erreicht werden kann. Insbesondere sollte die Konzentration so gewählt sein, daß - wie zuvor erwähnt - in der kontinuierlichen Phase des nichtverdünnten Wasch- oder Reinigungsmittels weniger als 1 % gelöstes Tensid vorliegt und bei Temperaturabsenkung, insbesondere bei Temperaturabsenkungen bis auf 0 °C, keine Auskristallisation des Sulfats stattfindet.

Da insbesondere nichtionische Tenside hinsichtlich der Stabilität von Peroxycarbonsäure problematisch sind, weisen die erfindungsgemäßen Wasch- und Reinigungsmittel ein entsprechend angepaßtes bzw. optimiertes Niotensid/geladenes Tensidverhältnis auf. Dabei sollte der Gehalt an Alkylpolyglykolethern möglichst gering sein.

Im Rahmen der vorliegenden Erfindung sollten die erfindungsgemäßen Wasch- und Reinigungsmittel, insbesondere flüssigen Wasch- und Reinigungsmittel, zumindest im wesentlichen keine Halogenidionen, insbesondere Chloridionen, aufweisen. Vorzugsweise beträgt die Menge an Halogenidionen, insbesondere keine Chloridionen, höchstens 500 ppm, vorzugsweise höchstens 100 ppm, besonders bevorzugt höchstens 30 ppm. Denn die Anmelderin hat überraschenderweise herausgefunden, daß eine hohe Halogenid-, insbesondere Chloridionenkonzentration, wie sie beispielsweise in herkömmlichen Wasch- und Reinigungsmitteln infolge von Verunreinigungen mancher Roh- bzw. Inhaltstoffe üblich ist, zu einem verstärkten Abbau von Peroxycarbonsäuren führt. Somit kann eine Verringerung der Halogenid-, insbesondere Chloridionenkonzentration, zu einem verminderten Abbau der Peroxycarbonsäure führen. Eine geringe Chloridionenkonzentration kann erfindungsgemäß insbesondere durch den Einsatz von Sulfat-, Methylsulfat-, Phosphat-, Tosylat- oder Cumolsulfonatverbindungen, insbesondere in bezug auf die Tenside der Wasch- oder Reinigungsmittelformulierung, erreicht werden. Ferner sollten Roh- bzw. Inhaltsstoffe ausgewählt werden, die einen besonders geringen Chloridgehalt aufweisen (z. B. Verwendung von im wesentlichen halogenidfreien Komponenten, so z. B. halogenidfreien Tensiden, halogenidfreien Phosphonaten).

Weiterhin sollten die erfindungsgemäßen Wasch- und Reinigungsmittel einen pH-Wert von höchstens 7, insbesondere einen pH-Wert von 3,5 bis 7, vorzugsweise von 4,0 bis 6,5, besonders bevorzugt von 4,5 bis 6, ganz besonders bevorzugt von etwa 5, aufweisen. Denn Bleichmittel auf Basis von Peroxycarbonsäuren, wie PAP, können überraschenderweise in einer sauren Umgebung, insbesondere bei einem pH-Wert ≤ 3,5, relativ wirkungsvoll stabilisiert werden, wohingegen bei neutralen oder alkalischen pH-Werten eine relativ schnelle Zersetzung von Peroxycarbonsäuren, wie PAP, stattfindet. Die Herabsetzung des pH-Wertes in den erfindungsgemäßen Wasch- und Reinigungsmitteln kann beispielsweise durch Zugabe saurer Salze erfolgen. Bevorzugt sind Bisulfate und/oder Bicarbonate oder organische Polycarbonsäuren, die z. B. gleichzeitig auch als Buildersubstanzen eingesetzt werden können. Ferner können die als Komplexbildner eingesetzten Phosphonate als Phosphonsäuren eingearbeitet werden und anschließend der gewünschte pH-Wert durch Zugabe von Alkalien eingestellt werden.

Die erfindungsgemäßen Wasch- oder Reinigungsmittel können mindestens eine Fettsäure enthalten. Dabei sind gesättigte und/oder verzweigte Fettsäuren, insbesondere mit einem Schmelzpunkt unterhalb von 30 °C, erfindungsgemäß bevorzugt. Im Rahmen der vorliegenden Erfindung kann beispielsweise Isocarb-16^{®} der Firma Sasol in den erfindungsgemäßen Wasch- oder Reinigungsmitteln eingesetzt werden.

Die erfindungsgemäßen Wasch- oder Reinigungsmittel weisen einen optimierten Citronensäure- bzw. Citratgehalt auf. Wie die Anmelderin herausgefunden hat, kann Citronensäure bzw. Citrat zu einem Abbau von Peroxycarbonsäuren, insbesondere PAP, führen. Dennoch kann es gegebenenfalls erforderlich sein, Citronensäure bzw. Citrate in dem Wasch- oder Reinigungsmittel bzw. in dem Dispersionsmittel für die erfindungsgemäßen Kapseln einzusetzen (zum Beispiel als Builder und/oder als Komplexbildner). Die verwendeten Mengen sollten dabei jedoch nicht zu hoch sein und in bezug auf die Peroxycarbonsäuren, insbesondere PAP, angepaßt sein.

Zudem kann das erfindungsgemäße Wasch- oder Reinigungsmittel mindestens einen Komplexbildner enthalten, der insbesondere ausgewählt sein kann aus der Gruppe von Chinolin und/oder seinen Salzen, Mono- oder Polyphosphonsäuren, insbesondere 1-Hydroxyethtyliden-1,2-diphosphonsäure (HEDP), Ethylendiamintetraessigsäure (EDTA), Diethylentriaminpenta(methylenphosphonsäure) (DTPMP) Azacycloheptandiphosphonat (AHP), Nitrilotriessigsäure (NTA), Citrat und/oder kurzkettige Dicarbonsäuren. Weitere Beispiele für erfindungsgemäß verwendbare Komplexbildner für Schwermetalle sind z. B. Aminopolycarbonsäuren, Aminohydroxypolycarbonsäuren, Polyphosphonsäuren und Aminopolyphosphonsäuren. Diese Komplexbildner werden erfindungsgemäß eingesetzt, um Schwermetallionen, die insbesondere als Katalysatoren von Oxidationsprozessen fungieren und somit zu einem Abbau von Peroxycarbonsäuren, wie PAP, führen können und die beispielsweise über Wasserleitungen oder metallische Bauteile der Produktionsanlagen oder auch über Roh- bzw. Inhaltsstoffe in Spuren in das erfindungsgemäße Wasch- oder Reinigungsmittel eingetragen werden können, wirkungsvoll zu inaktivieren bzw. zu binden.

Darüber hinaus können die erfindungsgemäßen Wasch- und Reinigungsmittel gegebenenfalls mindestens ein wassermischbares Lösemittel mit einem für die organische Peroxycarbonsäuren geringen Lösungsvermögen enthalten, wie vorzugsweise Glycerin.

Darüber hinaus können die erfindungsgemäßen Wasch- und Reinigungsmittel gegebenenfalls mindestens eine Katalase enthalten, um gegebenenfalls durch Umsetzung der Peroxycarbonsäure mit Wasser entstehendes Wasserstoffperoxid wirkungsvoll aus der kontinuierlichen Phase des Produktes, insbesondere der Wasch- und Reinigungsmittelformulierung, zu entfernen, so daß insbesondere dort gegebenenfalls vorhandene Enzyme wirkungsvoll vor Oxidationsprozessen, die gegebenenfalls zu einem Aktivitätsverlust der Enzyme führen können, geschützt werden. Zu diesem Zweck können den erfindungsgemäßen Wasch- oder Reinigungsmitteln gleichermaßen mindestens eine Peroxidase und/oder mindestens ein Antioxidans, gegebenenfalls zusätzlich zu der mindestens einen Katalase, zugegeben werden. Erfindungsgemäß bevorzugte Antioxidantien sind beispielsweise Ascorbinsäure, Tocopherol, Gallussäure oder deren Derivate.

Weiterhin sollte die erfindungsgemäße Wasch- oder Reinigungsmittelformulierung derart gestaltet sein, daß sie das erfindungsgemäße Kapselsystem insbesondere im wesentlichen nicht an- bzw. auflöst. Im allgemeinen sollten die Komponenten, welche in dem erfindungsgemäßen Wasch- oder Reinigungsmittel eingesetzt werden, derart ausgewählt sein, daß sie zumindest im wesentlichen kompatibel in bezug auf das erfindungsgemäße Kapselsystem sind, d. h. es sollten insbesondere in dem Wasch- oder Reinigungsmittel selbst, insbesondere in dem Zeitraum vor seiner Anwendung (Lagerzeit), keine unerwünschten chemischen Reaktionen, wie insbesondere Abbau-, Oxidations- bzw. Reduktionsreaktionen und/oder Hydrolysereaktionen, zwischen diesen Komponenten und dem Kapselsystem auftreten, welche zu einem vorzeitigen Abbau und einem Aktivitätsverlust der Peroxycarbonsäure führen.

Gleichzeitig sollte gewährleistet sein, daß in der Wasch- oder Reinigungsformulierung selbst keine Freisetzung der Peroxycarbonsäure erfolgt, insbesondere eine Abtrennung bzw. eine An- oder Auflösung der Kapselhülle bzw. der Hüllschichten nicht stattfindet bzw. die Peroxycarbonsäure sich aufgrund der osmotischen Verhältnisse nicht löst bzw. aus dem Kapselsystem diffundiert. Dies kann erfindungsgemäß in der Wasch- oder Reinigungsformulierung beispielsweise durch Zugabe von Sulfat in Kombination mit Wasser als Lösemittel, wodurch eine Erhöhung des osmotischen Drucks in der Wasch- oder Reinigungsformulierung erreicht wird, gewährleistet werden. Weiterhin sollte der pH-Wert der Wasch- oder Reinigungsformulierung im sauren Bereich liegen, insbesondere wie zuvor definiert, so daß die Peroxycarbonsäure, beispielsweise PAP, wenig löslich ist und andererseits gegebenenfalls vorhandene Hüllschichten des Kapselsystems mit von dem pH-Wert abhängigen kationischen Tensiden bzw. kationischen Polyelektrolyten, insbesondere wie oben definiert, protoniert vorliegen und daher eine nur geringe Wasserlöslichkeit aufweisen.

Die vorliegende Erfindung weist gegenüber dem Stand der Technik eine Reihe von Vorteilen auf:

Die Ausbildung der Kapselhülle bzw. der Hüllschichten erfolgt bei dem erfindungsgemäßen Verfahren aufgrund von physikalisch-chemischen bzw. physikalischen Wechselwirkungen, so daß für die Ausbildung der Kapselstruktur keine Polymerisationsschritte, insbesondere radikalische Polymerisationsschritte, notwendig sind, wie dies in einigen Verfahren des Standes der Technik der Fall ist. Derartige Polymerisationen führen häufig zur Zersetzung des Aktiv- und/oder Wirkstoffs, insbesondere der empfindlichen Peroxycarbonsäure. Durch die vorliegende Erfindung wird somit ein auf die chemisch empfindlichen Peroxycarbonsäuren ausgerichtetes Verkapselungsverfahren bereitgestellt.

Des weiteren hat das erfindungsgemäße Verfahren den Vorteil, daß es ein mit Peroxycarbonsäuren, insbesondere Imidoperoxycarbonsäuren, beladenes mehrschichtiges Kapselsystem bereitstellt, das hinsichtlich seiner Größe und seines Wirkstoffgehalts weit variiert bzw. maßgeschneidert werden kann, so daß eine individuelle Anpassung an die jeweiligen Anforderungen, insbesondere in bezug auf die Wasch- und Reinigungsmittel, erfolgen kann.

In diesem Zusammenhang ist es insbesondere vorteilhaft, daß sowohl die Anzahl der Kapselhüllen als auch die Zusammensetzung der entsprechenden Hüllen in einem weiten Bereich variiert werden kann, so daß es im Rahmen der vorliegenden Erfindung ermöglicht wird, die zu verkapselnde Peroxycarbonsäure einerseits mit einer hohen Lagerstabilität, insbesondere in bezug auf den Zeitraum vor ihrer Anwendung in der Waschflotte, zu versehen und andererseits eine gute Freisetzung der Peroxycarbonsäure während des Waschvorganges in der Waschflotte zu erreichen. Durch die Einstellbarkeit des Wirkstoff/Hülle-Verhältnisses wird eine effektive Dosierung des Wirkstoffes, d. h. der Peroxycarbonsäure, im Hinblick auf die entsprechende Anwendung möglich; dabei -zeichnet sich das erfindungsgemäße Kapselsystem durch einen hohen Gehalt an Peroxycarbonsäure aus.

Durch das erfindungsgemäße Herstellungsverfahren und das auf diese Weise erhältliche Kapselsystem ist gewährleistet, daß das erfindungsgemäße Kapselsystem einerseits aus wäßrigen Lösungen bzw. Dispersionen heraus aufgebracht werden kann und andererseits das Kapselsystem in einer wasserhaltigen flüssigen Formulierung, wie beispielsweise einem flüssigen Wasch- oder Reinigungsmittel, nicht an- bzw. aufgelöst wird. Dabei ist das erfindungsgemäße Verfahren zur Herstellung eines solchen Kapselsystems praktikabel und im industriell-technischen Maßstab realisierbar.

Weiterhin kann durch die gezielte Modifizierung der Kapselhülle, insbesondere durch die Zugabe von Komplexbildnern für Schwermetallionen, eine weitere Verbesserung des Schutzes der Peroxycarbonsäure und damit einhergehend eine weitere Erhöhung ihrer Lagerstabilität erreicht werden. Weitere Modifikationen der Kapselhülle, beispielsweise die Zugabe von Weichmachern, führt zu einer hervorragenden Anpassungsfähigkeit des erfindungsgemäßen Kapselsystems im Hinblick auf die jeweilige Anwendung.

Im Gegensatz zu Verkapselungssystemen, die beispielsweise auf Wachsen basieren, enthält das erfindungsgemäße Kapselsystem keine störenden Kapselhüllen, die innerhalb des Waschprozesses zu unerwünschten Rückständen auf der Wäsche führen. Denn obwohl die Grenzschichten zwischen den jeweiligen Hüllschichten im wesentlichen wasserunlöslich ausgebildet sein können, konnte die Anmelderin zeigen, daß sich unter Waschbedingungen keine nennenswerte Rückstande auf der Wäsche bilden.

Das erfindungsgemäße Kapselsystem weist den entscheidenden Vorteil auf, daß es gegenüber Systemen des Standes der Technik über eine deutliche Erhöhung der Lagerstabilität und damit auch nach längerer Zeit über eine hohe Bleichaktivität verfügt.

Insbesondere eignet sich das erfindungsgemäße Kapselsystem zur Einarbeitung bzw. Anwendung in Tenside enthaltenden Systemen, beispielsweise tensidischen (tensidhaltigen) Dispersionen für flüssige Wasch- und Reinigungsmittel. Dies ist ein besonderer Vorteil, da die unverkapselten bzw. ungeschützten Peroxycarbonsäuren, insbesondere PAP, in Gegenwart von Tensiden nicht stabil sind und rasch zersetzt werden, so daß ihr Einsatz in tensidhaltigen flüssigen, insbesondere wäßrigen Medien bislang nicht möglich bzw. allenfalls sehr beschränkt- möglich war. Der stabilisierende Effekt des Kapselsystems, der zusätzlich mit einer gewünschten kontrollierten Freisetzung der verkapselten Peroxycarbonsäure verbunden ist, kann in synergistischer Weise dadurch gesteigert werden, daß das Medium, in dem sich die erfindungsgemäßen Gelkapseln befinden, derart eingestellt wird, daß es eine zusätzliche Stabilisierung in bezug auf die Peroxycarbonsäuren liefert, insbesondere durch Inaktivierung der Tenside, Optimierung bzw. Absenkung des pH-Wertes, Reduzierung des Halogenidgehaltes, Verwendung eines Lösemittels mit geringem Lösungsvermögen in bezug aus Peroxycarbonsäuren und dergleichen.

Das erfindungsgemäße Kapselsystem läßt sich insbesondere in flüssige Wasch- und Reinigungsmittel stabil einarbeiten. Eine zusätzliche Verhinderung bzw. Verringerung von Sedimentationsprozessen kann beispielsweise durch geeignete, dem Fachmann an sich bekannte Verdickersysteme erreicht werden. Es verfügt dort über eine hohe Lagerstabilität und kann auch noch nach größeren Zeiträumen die Peroxycarbonsäure wirkungsvoll freisetzen.

Das erfindungsgemäße Kapselsystem ist in der erfindungsgemäßen Konfektionierungsform mit üblichen verfahrenstechnischen Möglichkeiten im technischen Maßstab herstellbar. Insbesondere können die einzelnen Hüllschichten direkt aufeinander aufgebracht werden, ohne daß zuvor nichtadsorbiertes Material im Rahmen eines aufwendigen Prozesses entfernt werden muß.

Mit Hilfe des erfindungsgemäßen Herstellungsverfahrens können Schichtdicken in einem großen Bereich ausgebildet werden, die signifikant dicker sind als eine molekulare Schicht, so daß eine aufeinanderfolgende bzw. alternierende Abfolge von komplexiereten und nichtkomplexierten Grenzschichten resultiert. Hieraus resultieren besonders positive Eigenschaften des erfindungsgemäßen Kapselsystems hinsichtlich der Lagerung und Freisetzung der Peroxycarbonsäure.

Die erfindungsgemäßen Wasch- und Reinigungsmittelformulierungen, welche das erfindungsgemäße Kapselsystem enthalten, weisen aufgrund ihrer zuvor angeführten, aufeinander abgestimmten und synergistisch wirkenden Modifikationen, d.h. Anpassung der Formulierung, _wie_insbesondere geringer Halogenidiongehalt, Optimierung des pH-Wertes, Zugabe von Komplexbildnern, speziellen Lösemitteln (z. B. Glycerin) und/oder Enzymen (z. B. Katalase), Inaktivierung der Tenside, gegenüber dem Stand der Technik erhebliche Vorteile auf, da in Verbindung mit dem erfindungsgemäßen Kapselsystem der Abbau der empfindlichen Bleichmittel auf Peroxycarbonsäurebasis deutlich vermindert wird.

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne daß er dabei den Rahmen der vorliegenden Erfindung verläßt

Die vorliegende Erfindung wird anhand der folgenden Ausführungsbeispiele verdeutlicht, welche die Erfindung jedoch keinesfalls beschränken:

### Ausführungsbeispiele:

### Beispiel 1:

In einer Labor-Wirbelschichtanlage (Aeromatic^{®}) wurden 300 g Eureco^{®} W (72 % PAP-Gehalt) vorgelegt. Bei einer Zulufttemperatur von 50 °C wurden darauf 150 ml Luviquat Care^{®} (Polykation) aufgesprüht, das mit 1 % Sequion^{®} 10H60 versetzt war. Nach Trocknen wurden 100 ml Sokalan^{®} CP 45 (Polyanion), wiederum versetzt mit 1 % Sequion^{®} 10H60, aufgesprüht. In gleicher Weise wurde eine dritte und eine vierte Schicht aufgesprüht. Das erhaltene Kapselsystem (Compound) wurde entnommen und auf eine Teilchengröße von 200 bis 2.000 µm abgesiebt. Es wies einen Aktivstoffgehalt (PAP) von 61 % auf.

### Beispiel 2:

Im folgenden Beispiel wird gezeigt, wie die erfindungsgemäße Konfektionierung die PAP-Stabilität in einer tensidhaltigen Flüssigformulierung erhöht:

Das Kapselsystem aus Beispiel 1 wurde in eine Flüssigformulierung folgender Zusammensetzung gebracht (die Prozentwerte sind Aktivstoffangaben):

| | |
|---|---|
| LAS (Maranil^{®} A 55) | 22,5 % |
| Dehydol^{®} LT 7 (Fa. Cognis) | 4 % |
| Sequion^{®} 10 H 60 (Fa. Polygon Chemie AG) | 1 % |
| Natriumsulfat | 12,5 % |
| Paraffin-Entschäumer | 0,6 % |
| Xanthan-Gummi | 1 % |
| Kapselsystem aus Beispiel 1 | 4 % |
| Wasser | ad 100 % |

Der pH-Wert dieser Formulierung ist aufgrund des Phosphonats (Sequion^{®} 10 H 60) stark sauer. Er wurde mit Natronlauge auf 5,0 eingestellt. Das Produkt wurde bei einer Temperatur von 40 °C eingelagert. Als Vergleichsbeispiel diente dieselbe Flüssigrezeptur, in der unkonfektioniertes Eureco^{®} W eingelagert wurde. Nach einer Woche betrug der Aktivsauerstoffverlust der erfindungsgemäßen

Rezeptur nur 1 %. Im Vergleichsbeispiel war 15 % des PAP abgebaut. Nach zwei Wochen wies die erfindungsgemäße Formulierung einen Verlust von nur 5 % auf. Im Vergleichsbeispiel sank der Gehalt um 25 % ab.

### Beispiel 3:

Rezeptur für eine weitere Flüssigformulierung, in der ein erfindungsgemäßes Kapselsystem eingelagert wird:

| | |
|---|---|
| LAS (Maranil^{®} A 55) | 18,5% |
| Dehydol^{®} LT 7 (Fa. Cognis) | 8 % |
| Natriumsulfat | 11 % |
| Xanthan-Gummi | 0,4 % |
| Sequion^{®} 10 H 60 (Fa. Polygon Chemie AG) | 1 % |
| Silikon-Entschäumer | 0,2 % |
| Kapselsystem aus Beispiel 1 | 3 % |
| Wasser | ad 100 % |

### Beispiel 4:

Die Kapselsysteme wurden gemäß Beispiel 1 hergestellt, wobei die Zusammensetzung der jeweiligen, zur Herstellung der Hüllschichten verwendeten Lösungen bzw. Dispersion variiert wurde. In den nachfolgenden Beispielen 4a bis 4d sind die für die jeweilige Lösung bzw. Dispersion des Hüllschichtmaterials verwendeten Materialien sowie deren Konzentrationen, bezogen auf die Lösung bzw. Dispersion (sofern nicht anders angeführt), angegeben. Die jeweiligen Lösungen bzw. Dispersionen wurden alternierend auf das zu verkapselnde Eureco^{®} W aufgesprüht, so daß ein Kapselsystem mit alternierenden Hüllschichten entsteht.

Die Kapselsysteme wurden in eine Flüssigformulierung gemäß Beispiel 3 gebracht und bei einer Lagertemperatur von 40 °C eingelagert. Angegeben sind die Aktivsauerstofferhaltungsgrade für verschiedene Einlagerungszeiten, bezogen auf den Aktivsauerstofferhaltungsgrad vor Einlagerung. Im Vergleich zu Beispiel 2 sind die Aktivsauerstofferhaltungsgrade etwas geringer, jedoch verfügt die verwendete Rezeptur über eine signifikant bessere Waschleistung.
4a: Beispiel für ein Kapselsystem mit 8 Hüllschichten:

| | | | |
|---|---|---|---|
| 4 Hüllschichten: | 1 % | Luviquat Care^{®} | |
| 4 Hüllschichten: | 3 % | Sokalan^{®} CP 45 | |
| 1. Woche | 2. Woche | 4. Woche | 8. Woche |
| 97,5% | 90,8% | 81,3% | 64,0% |

4b: Beispiel für ein Kapselsystem mit Abpuderungsmittel:

| | | |
|---|---|---|
| 4 Hüllschichten: | 2 % Luviquat Care^{®} auf Luviquat Care^{®}) | (mit 10 % Sipemat^{®} S22, bezogen |
| 4 Hüllschichten: | 5 % Sokalan^{®} CP 45 | |
| 1. Woche | 2. Woche | |
| 95,1% | 89,4 % | |

4c: 4 üllschichten: 3 % Luviquat^{®} PQ 11 N

| | | | |
|---|---|---|---|
| 4 Hüllschichten: | 5% | Sokalan^{®} CP 45 | |
| 1. Woche | 2. Woche | 4. Woche | 8. Woche |
| 95,4% | 89,7% | 82,4% | 64,0% |

4d: 4 Hüllschichten: 3 % Polyvinylpyrrolidon (PVP)

| | | |
|---|---|---|
| 4 Hüllschichten: | 5 % | Sokalan^{®} CP 45 |
| Der pH-Wert | der | Polyvinylpyrrolidonlösung betrug 4,0 (Einstellung mit Schwefelsäure). |
| 1. Woche | 2. Woche | |
| 95,3% | 89,7 % | |

4e (Vergleichsbeispiel):
Unkonfektioniertes Eureco^{®} W ("ungecoalete PAP") in derselben Rezeptur

| | | | |
|---|---|---|---|
| 1. Woche | 2. Wochen | 4. Woche | 8. Woche |
| 85,7% | 76,9 % | 63,5 % | 41,5 % |

## Patentansprüche

1. Verfahren zur Herstellung eines mit mindestens einer organischen Peroxycarbonsäure, insbesondere Imidoperoxycarbonsäure, beladenen mehrschichtigen Kapselsystems, **dadurch gekennzeichnet, daß** auf eine in Form fester Teilchen vorliegende organische Peroxycarbonsäure, insbesondere Imidoperoxycarbonsäure, aufeinanderfolgend mindestens zwei voneinander verschiedene Hüllschichten jeweils auf Basis mindestens eines Polyelektrolyten und/oder ionischen Tensids aufgebracht werden, so daß ein Kapselsystem resultiert, welches mindestens eine organische Peroxycarbonsäure, insbesondere Imidoperoxycarbonsäure, in einer mehrschichtigen Kapselhülle aus mindestens zwei Hüllschichten umfaßt, umfassend die folgenden Verfahrensschritte:
(a) Aufbringen einer mindestens einen ersten Polyelektrolyten und/oder mindestens ein erstes ionisches Tensid (I) enthaltenden Lösung und/oder Dispersion auf mindestens eine in Form fester Teilchen vorliegende organische Peroxycarbonsäure, insbesondere Imidoperoxycarbonsäure, so daß die Peroxycarbonsäure von einer ersten Hüllschicht auf Basis des ersten Polyelektrolyten und/oder des ersten ionischen Tensids (I) vollständig umhüllt bzw. beschichtet wird; dann
(b) Aufbringen mindestens einer einen zweiten Polyelektrolyten und/oder mindestens ein zweites ionisches Tensid (II) enthaltenden Lösung und/oder Dispersion auf die in Verfahrensschritt (a) erhaltene erste Hüllschicht, wobei der zweite Polyelektrolyt und/oder das zweite ionische Tensid (II) von dem ersten Polyelektrolyten und/oder ersten ionischen Tensid (I) verschieden und entgegengesetzt geladen ist, so daß eine zweite Hüllschicht auf Basis des zweiten Polyelektrolyten und/oder des zweiten ionischen Tensids (II) auf die in Verfahrensschritt (a) erhaltene erste Hüllschicht aufgebracht wird, wobei die zweite Hüllschicht die erste Hüllschicht vollständig umhüllt bzw. beschichtet und die beiden Hüllschichten in direktem Kontakt zueinander sind; dann
(c) gegebenenfalls Aufbringen mindestens einer weiteren Hüllschicht, insbesondere einer dritten, vierten etc. Hüllschicht, wobei die die Hüllschicht(en) ausbildenden Polyelektrolyte und/oder ionischen Tenside derart ausgewählt werden, daß jeweils unmittelbar aneinandergrenzende Hüllschichten voneinander verschiedene, insbesondere entgegengesetzt geladene Polyelektrolyte und/oder entgegengesetzt geladenen ionische Tenside umfassen oder hieraus bestehen; anschließend
(d) gegebenenfalls Aufbereiten, insbesondere Trocknen und/oder Aufreinigen und/oder Klassieren, insbesondere Sieben, des erhaltenen Kapselsystems,
wobei der Polyelektrolyt und/oder das ionische Tensid der in Verfahrensschritt (a) erhaltenen ersten Hüllschicht und der gegebenenfalls in Verfahrenschritt (c) erhaltenen dritten, fünften etc. Hüllschicht eine positive Nettoladung aufweist, und die erste Hüllschicht durch ein kationisches Tensid oder einen kationischen Polyelektrolyten, vorzugsweise durch ein kationisches Tensid, gebildet wird und die gegebenenfalls vorhandene dritte, fünfte etc. Hüllschicht durch einen kationischen Polyelektrolyten (Polykation) gebildet wird, und daß der Polyelektrolyt und/oder das ionische Tensid der in Verfahrensschritt (b) erhaltenen zweiten Hüllschicht und der gegebenenfalls in Verfahrenschritt (c) erhaltenen vierten, sechsten etc. Hüllschicht eine negative Nettoladung aufweist, insbesondere wobei der Polyelektrolyt der zweiten und gegebenenfalls vierten, sechsten etc. Hüllschicht ein anionischer Polyelektrolyt (Polyanion) ist und/oder daß die äußerste Hüllschicht vorzugsweise mindestens einen anionischen Polyelektrolyten aufweist oder hieraus besteht.

2. Verfahren nach Anspruch 1, **dadurch gekennzeichnet, daß** die organische Peroxycarbonsäure ausgewählt ist aus organischen Mono- und Diperoxycarbonsäuren, insbesondere Dodekandiperoxysäure oder vorzugsweise Imidoperoxycarbonsäuren, besonders bevorzugt 6-Phthalimidoperoxycapronsäure (6-Phthalimidoperoxyhexansäure, PAP), und/oder daß die Peroxycarbonsäure bei Atmosphärendruck einen Schmelzpunkt oberhalb von 25 °C, insbesondere oberhalb von 35 °C, vorzugsweise oberhalb von 45 °C, bevorzugt oberhalb von 50 °C, besonders bevorzugt oberhalb von 100 °C, aufweist.

3. Verfahren nach einem oder mehreren der vorangehenden Ansprüche, **dadurch gekennzeichnet, daß** die erste Hüllschicht vorzugsweise mindestens ein kationisches Tensid umfaßt und/oder daß die zweite Hüllschicht und die gegebenenfalls dritte, vierte etc. Hüllschicht mindestens ein ionisches Tensid oder eine Mischung aus mindestens einem Polyelektrolyten mit mindestens einem ionischen Tensid, wobei der mindestens eine Polyelektrolyt und das mindestens eine ionische Tensid eine gleichartige Nettoladung aufweisen, und besonders bevorzugt mindestens einen Polyelektrolyten umfaßt.

4. Verfahren nach einem oder mehreren der vorangehenden Ansprüche, **dadurch gekennzeichnet, daß** das kationische Tensid, insbesondere der ersten Hüllschicht, ausgewählt ist aus quaternären Ammoniumsalzen, deren Ammoniumrest der allgemeinen Formel R¹R²R³N⁺ entspricht, wobei die Reste R¹, R² und R³, identisch oder verschieden, ein Wasserstoffatom oder einen linearen oder verzweigten Alkyl-, Alkylen- oder Alkinylrest mit 1 bis 40 Kohlenstoffatomen, insbesondere 1 bis 25 Kohlenstoffatomen, darstellen, und/oder daß das kationische Tensid insbesondere ausgewählt ist aus der Gruppe von Alkyldimethylammoniumtensiden, N-Alkylpyridiniumsalzen und Esterquats, insbesondere wobei das kationische Tensid keine Halogenidionen, insbesondere keine Chloridionen, enthält oder zumindest im wesentlichen halogenidfrei, insbesondere chloridfrei, ist und vorzugsweise ausgewählt ist aus Methylsulfat-, Sulfat-, Phosphat-, Tosylat- oder Cumolsulfonatverbindungen.

5. Verfahren nach einem oder mehreren der vorangehenden Ansprüche, **dadurch gekennzeichnet, daß** der kationische Polyelektrolyt, insbesondere der ersten Hüllschicht, mindestens eine funktionelle Gruppe, ausgewählt aus quaternären Amin-, Imin- und Imidazolgruppen, umfaßt, und/oder daß der kationische Polyelektrolyt, insbesondere der ersten Hüllschicht, ausgewählt ist aus Aminoxiden, Pyridin-N-oxiden, vorzugsweise Polyvinylpyridin-N-oxid, insbesondere wobei der kationische Polyelektrolyt keine Halogenidionen, insbesondere keine Chloridionen, enthält oder zumindest im wesentlichen halogenidfrei, insbesondere chloridfrei, ist.

6. Verfahren nach einem oder mehreren der vorangehenden Ansprüche, **dadurch gekennzeichnet, daß** der anionische Polyelektrolyt, insbesondere der zweiten, vierten etc. Hüllschicht, ein synthetischer anionischer Polyelektrolyt ist, ausgewahlt aus der Gruppe von polymeren Sulfonsäuren, insbesondere Polystyrolsulfonsäuren und deren (partiellen) Salzen; vorzugsweise Polycarbonsäuren und deren (partiellen) Salzen, wie insbesondere Polyacrylsäure, Polymaleinsäure und deren Copolymeren, und/ oder daß der anionische Polyelektrolyt ein polymeranalog sulfoniertes Polymer ist und/oder daß der anionische Polyelektrolyt ein natürliches anionisches Polymer ist, ausgewählt aus der Gruppe von Alginsäure, Xanthan und/oder derivatisierten natürlichen Polymeren, wie Carboxymethylcellulose.

7. Verfahren nach einem oder mehreren der vorangehenden Ansprüche, **dadurch gekennzeichnet, daß** der anionische Polyelektrolyt, insbesondere der zweiten, vierten etc. Hüllschicht, ein natürliches anionisches Polymer ist, insbesondere ausgewählt aus der Gruppe von Alginat, Carboxymethylamylose, Carboxymethylcellulose, Carboxymethyldextran, Carageenan, Cellulosesulfat, Chondroitinsulfat, Chitosansulfat, Dextransulfat, Gummi Arabicum, Gummi Guar, Gummi Gellan, Heparin, Hyaluronsäure, Pektin, Xanthan und anionischen Proteinen, und/oder daß der Polyelektrolyt ein synthetischer anionischer Polyelektrolyt ist, insbesondere ausgewählt aus der Gruppe von Polyacrylaten, anionischen Polyaminosauren und deren Copolymeren, Polymaleinat, Polymethacrylat, Polystyrolsulfat, Polystyrolsulfonat, Polyvinylphosphat, Polyvinylphosphonat, Polyvinylsulfat, Polyacrylamidmethylpropansulfonat, Polylactat, Poly-(Butadien/Maleinat), Poly-(Ethylen/Maleinat), Poly-(Ethacrylat/Acrylat) und Poly-(Glycerin/Methacrylat).

8. Verfahren nach einem oder mehreren der vorangehenden Ansprüche, **dadurch gekennzeichnet, daß** der kationische Polyelektrolyt, insbesondere der dritten, fünften etc. Hüllschicht, ein natürlicher kationischer Polyelektrolyt oder ein modifizierter, natürlicher kationischer Polyelektrolyt ist, insbesondere ausgewählt aus der Gruppe von Chitosan, modifizierten Dextranen, wie diethylaminoethylmodifizierten Dextranen, Hydroxymethylcellulosetrimethylamin, Lysozym, Polylysin, Protaminsulfat, Hydroxyethylcellulosetrimethylamin und kationischen Proteinen, und/oder daß der kationische Polyelektrolyt ein synthetischer kationischer Polyelektrolyt ist, insbesondere ausgewählt aus der Gruppe von Polyallylamin, Polyallylaminhydrosalzen, Polyaminen, Polyvinylbenzyltrimethylammoniumsalzen, Polybren, Polydiallyldimethylammoniumsalzen, Polyethylenimin, Polyimidazolin, Polyvinylamin, Polyvinylpyridin, Poly-(Acrylamid/Methacryloxypropyltrimethylammoniumsalzen), Poly-(Diallyldimethylammoniumsalzen/N-Isopropylacrylamid), Poly-(Dimethylaminoethylacrylat/Acrylamid), Polydimethylaminoethylmethacrylat, Polydimethylaminoepichlorhydrin, Polyethyleniminoepichlorhydrin, Polymethacryloxyethyltrimethylammoniumsalzen, Hydroxypropylmethacryloxyethyldimethylammoniumsalzen, Poly-(Methyldiethylaminoethylmethacrylat/Acrylamid), Poly-(Methyl/Guanidin), Polymethylvinylpyridiniumsalzen, Poly-(Vinylpyrrolidon/Dimethylaminoethylmethacrylat) und Polyvinylmethylpyridiniumsalzen, insbesondere wobei der kationische Polyelektrolyt im wesentlichen keine Halogenidionen, insbesondere keine Chloridionen, enthält oder zumindest im wesentlichen halogenidfrei, insbesondere chloridfrei, vorliegt.

9. Verfahren nach einem oder mehreren der vorangehenden Ansprüche, **dadurch gekennzeichnet, daß** das kationische Tensid, insbesondere der ersten, dritten, fünften etc. Hüllschicht, ein von dem pH-Wert, insbesondere von dem pH-Wert des Dispersionsmittels, abhängiges kationisches Tensid ist, insbesondere wobei das von dem pH-Wert abhängige kationische Tensid bei einem sauren pH-Wert, bevorzugt bei einem pH-Wert kleiner als 7, insbesondere bei einem pH-Wert kleiner als 6,5, vorzugsweise kleiner als 6, zumindest im wesentlichen protoniert und/oder kationisch vorliegt und/oder das von dem pH-Wert abhängige kationische Tensid bei einem neutralen oder alkalischen pH-Wert zumindest im wesentlichen deprotoniert und/oder elektrisch neutral vorliegt, und/oder daß der kationische Polyelektrolyt ein von dem pH-Wert, insbesondere von dem pH-Wert des Dispersionsmittels, abhängiger kationischer Polyelektrolyt ist, insbesondere wobei der von dem pH-Wert abhängige kationische Polyelektrolyt bei einem sauren pH-Wert, bevorzugt bei einem pH-Wert kleiner als 7, insbesondere bei einem pH-Wert kleiner als 6,5, vorzugsweise kleiner als 6, zumindest im wesentlichen protoniert und/oder kationisch vorliegt, und/oder insbesondere wobei der von dem pH-Wert abhängige kationische Polyelektrolyt, mindestens eine funktionelle Gruppe, ausgewählt aus Amino-, Imino-, Aminoxid-, Phosphinoxid- und Pyridin-N-oxidgruppen, vorzugsweise Aminoxid-, Phosphinoxid-, Pyridin-N-oxid- und Pyridiniumgruppen, aufweist.

10. Verfahren nach einem oder mehreren der vorangehenden Ansprüche, **dadurch gekennzeichnet, daß** in zwischen den jeweiligen Hüllschichten ausgebildeten Grenzschichten Grenzschicht-Komplexe, insbesondere in Form von Polykation/Polyanion-, Kationtensid/Polyanion-, Polykation/Anlontensid- und/oder Aniontensid/Kationtensid-Komplexen, ausgebildet werden, insbesondere wobei die Grenzschicht-Komplexe in Abhängigkeit von der Stöchiometrie wasserlöslich, mäßig wasserlöslich oder wasserunlöslich ausgebildet sind und/oder insbesondere wobei die Wasserlöslichkeit der Grenzschicht-Komplexe von dem pH-Wert, insbesondere von dem pH-Wert des Dispersionsmittels, abhängen.

11. Verfahren nach einem oder mehreren der vorangehenden Ansprüche, **dadurch gekennzeichnet, daß** auf die mindestens eine organische Peroxycarbonsäure, insbesondere Imidoperoxycarbonsäure, eine Vielzahl von Hüllschichten, insbesondere von mindestens zwei und bis zu zehn oder mehr Hüllschichten aufgebracht werden.

12. Verfahren nach einem oder mehreren der vorangehenden Ansprüche, **dadurch gekennzeichnet, daß** die in Verfahrenschritt (a), (b) und/oder gegebenenfalls (c) bereitgestellten, mindestens einen Polyelektrolyten und/oder mindestens ein ionisches Tensid enthaltenden Lösungen und/oder Dispersionen einen pH-Wert von höchstens 6, insbesondere einen pH-Wert von 1 bis 6, vorzugsweise von 2 bis 5, bevorzugt von 3 bis 4, besonders bevorzugt von etwa 3,5, aufweisen.

13. Verfahren nach einem oder mehreren der vorangehenden Ansprüche, **dadurch gekennzeichnet, daß** in Verfahrensschritt (a), (b) und/oder gegebenenfalls (c) das Aufbringen der Lösung und/oder Dispersion auf die zu stabilisierende Peroxycarbonsäure und/oder auf die vorangehende Hüllschicht durch Dragierkessel, Trommelcoater, Mischer, insbesondere Sprühtrockner, Wurstercoater, vorzugsweise durch Aufsprühen der Lösung und/oder Dispersion in einer Wirbelschichtanlage, erfolgt.

14. Verfahren nach einem oder mehreren der vorangehenden Ansprüche, **dadurch gekennzeichnet, daß** mindestens einer Hüllschicht mindestens ein Komplexbildner, insbesondere ausgewählt aus der Gruppe von Chinolin und/oder seinen Salzen, Phosphaten, Alkalimetallpolyphosphonaten, Picolinsäure und Dipicolinsäure, Mono- oder Potyphosphonsäuren, insbesondere 1-Hydroxyethyliden-1,1-diphosphonsäure (HEDP), Ethylendiamintetraessigsäure (EDTA), Diethylentriaminpenta(methylenphosphonsäure) (DTPMP), Azacycloheptandiphosphonat (AHP) und/oder Nitrilotriessigsäure (NTA), insbesondere zur Komplexierung von Schwermetallionen, zugegeben wird und/oder daß mindestens einer Hüllschicht mindestens ein Weichmacher, insbesondere mindestens ein Weichmacher für wasserlösliche Polymere, vorzugsweise Polyethylenglykol, Glycerin, Glykol oder Triacetin, zugegeben wird.

15. Verfahren nach einem oder mehreren der vorangehenden Ansprüche, **dadurch gekennzeichnet, daß** in Verfahrensschritt (a), (b) und/oder gegebenenfalls (c) ein Aufbereiten, insbesondere ein Trocknen und/oder ein Aufreinigen und/oder ein Klassieren des Kapselsystems, insbesondere nach Aufbringen der jeweiligen Hüllschicht, durch übliche Methoden erfolgt, insbesondere durch Gefriertrocknung (Lyophilisierung), Verdampfen des Dispersiansmittels, vorzugsweise bei einer Temperatur von 40 °C bis 60 °C in einer Wirbelschichtanlage, Ultrafiltration, Dialyse oder Sprühtrocknung unter schonenden Bedingungen, Sieben.

16. Verfahren nach einem oder mehreren der vorangehenden Ansprüche, **dadurch gekennzeichnet, daß** das in Verfahrensschritt (d) gegebenenfalls durchgeführte Aufbereiten, insbesondere Trocknen und/oder Aufreinigen und/oder Klassieren, durch übliche Methoden, insbesondere wie in Anspruch 15 definiert, erfolgt.

17. Verfahren nach einem oder mehreren der vorangehenden Ansprüche, **dadurch gekennzeichnet, daß** das mit mindestens einer organischen Peroxycarbonsäure, insbesondere Imidoperoxycarbonsäure, beladene Kapselsystem mit einer mittleren Größe (Kugeldurchmesser) von 20 µm bis 4.000 µm, vorzugsweise 50 µm bis 3.000 µm, bevorzugt 100 µm bis 2.000 µm, erhalten wird.

18. Verfahren nach einem oder mehreren der vorangehenden Ansprüche, **dadurch gekennzeichnet, daß** der Anteil der jeweiligen Hüllschicht der Kapselhülle 1 bis 15 Gew.-%, vorzugsweise 1,5 bis 10 Gew.-%, bevorzugt 2 bis 10 Gew.-%, bezogen auf das Kapselsystem, beträgt und/oder daß das Molekulargewicht des Polyelektrolyten in der jeweiligen Hüllschicht ≥ 1.000, vorzugsweise ≥ 10.000, bevorzugt ≥ 15.000, ist und/oder daß der Gehalt an organischer Peroxycarbonsäure, insbesondere Imidoperoxycarbonsäure, vorzugsweise PAP, ≥ 30 Gew.-%, vorzugsweise ≥ 40 Gew.-%, bevorzugt ≥ 50 Gew.-%, bezogen auf das Kapselsystem, beträgt.

19. Verfahren nach einem oder mehreren der vorangehenden Ansprüche, **dadurch gekennzeichnet, daß** das in Verfahrensschritt (b), (c) und/oder gegebenenfalls (d) erhaltene Kapselsystem zusammen mit weiteren Inhaltsstoffen zu einem Wasch- oder Reinigungsmittel, insbesondere einem flüssigen Wasch- oder Reinigungsmittel, formuliert wird, insbesondere wobei das Wasch- oder Reinigungsmittel:
- zumindest im wesentlichen keine Halogenidionen, insbesondere keine Chloridionen, aufweist und die Menge an Halogenidionen, insbesondere Chloridionen, höchstens 500 ppm, vorzugsweise höchstens 100 ppm, besonders bevorzugt höchstens 30 ppm, beträgt; und/oder
- einen pH-Wert von höchstens 7, insbesondere einen pH-Wert von 3,5 bis 7, vorzugsweise von 4,0 bis 6,5, besonders bevorzugt von 4,5 bis 6, ganz besonders bevorzugt von etwa 5, aufweist; und/oder
- mindestens einen Komplexbildner enthält, insbesondere ausgewählt aus der Gruppe von Chinolin und/oder seinen Salzen, Alkalimetallpolyphosphonaten, Picolinsäure und Dipicolinsäure, Mono- oder Polyphosphonsäuren, insbesondere 1-Hydroxyethyliden-1,1-diphosphonsäure (HEDP), Ethylendiamintetraessigsäure (EDTA), Diethylentriaminpenta(methylenphosphonsäure) (DTPMP) Azacycloheptandiphosphonat (AHP), Nitrilotriessigsäure (NTA), Citrat und/oder kurzkettige Dicarbonsäuren, insbesondere zur Komplexierung von Schwermetallionen; und/oder
- gegebenenfalls mindestens ein wassermischbares Lösemittel mit einem für die organische Peroxycarbonsäure, insbesondere Imidoperoxycarbonsäure, geringen Lösungsvermögen, vorzugsweise Glycerin, enthält; und/oder
- gegebenenfalls mindestens ein Enzym, insbesondere mindestens eine Katalase und/oder mindestens eine Peroxidase, vorzugsweise mindestens eine Katalase, und/oder mindestens ein Antioxidans enthält.

20. Verfahren nach einem der Ansprüche 1 bis 19 zur Herstellung eines mit mindestens einer Imidoperoxycarbonsäure, vorzugsweise PAP, beladenen mehrschichtigen Kapselsystems.

21. Verfahren nach einem der Ansprüche 1 bis 20 zur Stabilisierung von Peroxycarbonsäuren, insbesondere Imidoperoxycarbonsäuren, vorzugsweise PAP, und/oder zur Erhöhung der Lagerfähigkeit von Peroxycarbonsäuren, insbesondere Imidoperoxycarbonsäuren, vorzugsweise PAP.

22. Kapselsystem, erhältlich nach dem Verfahren gemäß den Ansprüchen 1 bis 21.

23. Tensidische Dispersionen, insbesondere wäßrige Dispersionen, enthaltend das Kapselsystem nach Anspruch 22.

24. Verwendung des Kapselsystems nach Anspruch 22 und/oder der Dispersionen nach Anspruch 23 für Wasch- und Reinigungsmittel, insbesondere flüssige Wasch- und Reinigungsmittelzusammensetzungen, Zahnpflegemittel, Haarfärbemittel oder für Entfärbungs- bzw. Bleichmittelzusammensetzungen für technische Anwendungen.

25. Wasch- und Reinigungsmittel, insbesondere flüssige Wasch- und Reinigungsmittelzusammensetzungen, Zahnpflegemittel, Haarfärbemittel oder Entfärbungs- bzw. Bleichmittel für technische Anwendungen, enthaltend das Kapselsystem nach Anspruch 22 und/oder Dispersionen nach Anspruch 23.

## Claims

1. A method for preparing a multilayer capsule system loaded with at least one organic peroxycarboxylic acid, in particular imidoperoxycarboxylic acid, **characterized in that** at least two coating layers that are different from one another and are in each case based on at least one polyelectrolyte and/or ionic surfactant are applied successively to an organic peroxycarboxylic acid, in particular imidoperoxycarboxylic acid, that is present in the form of solid particles, resulting in a capsule system that comprises at least one organic peroxycarboxylic acid, in particular imidoperoxycarboxylic acid, in a multilayer capsule coating made of at least two coating layers, comprising the following method steps:
(a) applying a solution and/or dispersion containing at least one first polyelectrolyte and/or at least one first ionic surfactant (I) to at least one organic peroxycarboxylic acid, in particular imidoperoxycarboxylic acid, that is present in the form of solid particles, such that the peroxycarboxylic acid is completely encased or coated by a first coating layer based on the first polyelectrolyte and/or the first ionic surfactant (I); then
(b) applying at least one solution and/or dispersion containing a second polyelectrolyte and/or at least one second ionic surfactant (II) to the first coating layer obtained in method step (a), the second polyelectrolyte and/or the second ionic surfactant (II) being different from and being loaded differently compared with the first polyelectrolyte and/or the first ionic surfactant (I), such that a second coating layer based on the second polyelectrolyte and/or the second ionic surfactant (II) is applied to the first coating layer obtained in method step (a), the second coating layer completely encasing or coating the first coating layer and the two coating layers being in direct contact with one another; then
(c) optionally applying at least one additional coating layer, in particular a third, fourth, etc., coating layer, the polyelectrolytes and/or ionic surfactants forming the coating layer(s) being selected such that in each case directly adjacent coating layers comprise or consist of polyelectrolytes that are different from one another, in particular loaded differently, and/or differently loaded ionic surfactants; subsequently
(d) optionally processing, in particular drying and/or cleaning and/or sizing, in particular sieving the obtained capsule system,
the polyelectrolyte and/or the ionic surfactant of the first coating layer obtained in method step (a) and the third, fifth, etc., coating layer optionally obtained in method step (c) having a positive net charge, and the first coating layer being formed by a cationic surfactant or a cationic polyelectrolyte, preferably by a cationic surfactant, and the optionally present third, fifth, etc., coating layer being formed by a cationic polyelectrolyte (polycation), and **in that** the polyelectrolyte and/or the ionic surfactant of the second coating layer obtained in method step (b) and the fourth, sixth, etc., coating layer optionally obtained in method step (c) have a negative net charge, in particular the polyelectrolyte of the second and optionally fourth, sixth, etc., coating layer being an anionic polyelectrolyte (polyanion) and/or **in that** the outermost coating layer preferably comprises or consists of at least one anionic polyelectrolyte.

2. The method according to claim 1, **characterized in that** the organic peroxycarboxylic acid is selected from organic mono- and diperoxycarboxylic acids, in particular dodecanediperoxy acid or preferably imidoperoxycarboxylic acids, particularly preferably 6-phthalimidoperoxycaproic acid (6-phthalimidoperoxyhexanoic acid, PAP), and/or **in that** the peroxycarboxylic acid has, at atmospheric pressure, a melting point higher than 25 °C, in particular higher than 35 °C, preferably higher than 45 °C, preferably higher than 50 °C, particularly preferably higher than 100 °C.

3. The method according to one or more of the preceding claims, **characterized in that** the first coating layer preferably comprises at least one cationic surfactant and/or **in that** the second coating layer and the optionally third, fourth, etc., coating layer comprises at least one ionic surfactant or a mixture of at least one polyelectrolyte with at least one ionic surfactant, the at least one polyelectrolyte and the at least one ionic surfactant having a similar net charge, and particularly preferably at least one polyelectrolyte.

4. The method according to one or more of the preceding claims, **characterized in that** the cationic surfactant, in particular the first coating layer, is selected from quaternary ammonium salts of which the ammonium functional group corresponds to the general formula R¹R²R³N⁺, the functional groups R¹, R² and R³, which may be identical or different, representing a hydrogen atom or a linear or branched alkyl functional group, alkylene functional group or alkynyl functional group having from 1 to 40 carbon atoms, in particular from 1 to 25 carbon atoms, and/or **in that** the cationic surfactant is in particular selected from the group consisting of alkyldimethyl ammonium surfactants, N-alkylpyridinium salts and esterquats, in particular the cationic surfactant containing no halide ions, in particular no chloride ions, or being at least substantially halide-free, in particular chloride-free and preferably selected from methylsulfate, sulfate, phosphate, tosylate or cumene sulfonate compounds.

5. The method according to one or more of the preceding claims, **characterized in that** the cationic polyelectrolyte, in particular the first coating layer, comprises at least one functional group, selected from quaternary amine, imine and imidazole groups, and/or **in that** the cationic polyelectrolyte, in particular the first coating layer, is selected from amine oxides, pyridine-N-oxides, preferably polyvinylpyridine-N-oxide, in particular the cationic polyelectrolyte containing no halide ions, in particular no chloride ions or being at least substantially halide-free, in particular chloride-free.

6. The method according to one or more of the preceding claims, **characterized in that** the anionic polyelectrolyte, in particular the second, fourth, etc., coating layer, is a synthetic anionic polyelectrolyte, selected from the group consisting of polymeric sulfonic acids, in particular polystyrene sulfonic acids and (partial) salts thereof; preferably polycarboxylic acids and (partial) salts thereof, such as in particular polyacrylic acid, polymaleic acid and copolymers thereof, and/or in that the anionic polyelectrolyte is a polymer-like sulfonated polymer and/or **in that** the anionic polyelectrolyte is a natural anionic polymer, selected from the group consisting of alginic acid, xanthan gum and/or derivatized natural polymers such as carboxymethyl cellulose.

7. The method according to one or more of the preceding claims, **characterized in that** the anionic polyelectrolyte, in particular the second, fourth, etc., coating layer, is a natural anionic polymer, in particular selected from the group of alginate, carboxymethyl amylose, carboxymethyl cellulose, carboxymethyl dextran, carrageenan, cellulose sulfate, chondroitin sulfate, chitosan sulfate, dextran sulfate, gum arabic, guar gum, gellan gum, heparin, hyaluronic acid, pectin, xanthan gum and anionic proteins, and/or **in that** the polyelectrolyte is a synthetic anionic polyelectrolyte, in particular selected from the group consisting of polyacrylates, anionic polyamino acids and copolymers thereof, polymaleate, polymethacrylate, polystyrene sulfate, polystyrene sulfonate, polyvinyl phosphate, polyvinyl phosphonate, polyvinyl sulfate, polyacrylamidomethylpropanesulfonate, polylactate, poly (butadiene/maleate), poly (ethylene/maleate), poly(ethacrylate/acrylate) and poly(glycerol/methacrylate).

8. The method according to one or more of the preceding claims, **characterized in that** the cationic polyelectrolyte, in particular the third, fifth, etc., coating layer, is a natural cationic polyelectrolyte or a modified natural cationic polyelectrolyte, in particular selected from the group consisting of chitosan, modified dextrans, such as diethylaminoethyl-modified dextrans, hydroxymethylcellulosetrimethylamine, lysozyme, polylysine, protamine sulfate, hydroxyethylcellulosetrimethylamine and cationic proteins, and/or **in that** the cationic polyelectrolyte is a synthetic cationic polyelectrolyte, in particular selected from the group consisting of polyallylamine, polyallylamine hydrosalts, polyamines, polyvinylbenzyltrimethylammonium salts, polybrene, polydiallyidimethylammonium salts, polyethyleneimine, polyimidazoline, polyvinylamine, polyvinylpyridine, poly(acrylamide/methacryloxypropyltrimethylammonium salts), poly(diallyldimethylammonium salts/N-isopropylacrylamide), poly(dimethylaminoethyl acrylate/acrylamide), polydimethylaminoethyl methacrylate, polydimethylaminoepichlorohydrin, polyethyleneiminoepichlorohydrin, polymethacryloxyethyltrimethylammonium salts, hydroxypropylmethacryloxyethyldimethylammonium salts, poly(methyldiethylaminoethyl methacrylate/acrylamide), poly(methyl/guanidine), polymethylvinylpyridinium salts, poly(vinylpyrrolidone/dimethylaminoethyl methacrylate) and polyvinylmethylpyridinium salts, in particular the cationic polyelectrolyte containing substantially no halide ions, in particular no chloride ions, or being present in a form that is at least substantially halide-free, in particular chloride free.

9. The method according to one or more of the preceding claims, **characterized in that** the cationic surfactant, in particular the first, third, fifth, etc., coating layer, is a cationic surfactant that is dependent on the pH, in particular the pH of the dispersing agent, in particular the pH-dependent cationic surfactant, at an acidic pH, preferably at a pH less than 7, in particular at a pH less than 6.5, preferably less than 6, being present at least substantially in protonated form and/or in cationic form and/or the pH-dependent cationic surfactant, at a neutral or alkaline pH, being present at least substantially in deprotonated form and/or in electrically neutral form, and/or **in that** the cationic polyelectrolyte is a cationic polyelectrolyte that is dependent on the pH, in particular the pH of the dispersing agent, in particular the pH-dependent cationic polyelectrolyte, at an acidic pH, preferably at a pH less than 7, in particular at a pH less than 6.5, preferably less than 6, being present at least substantially in protonated form and/or in cationic form, and/or in particular the pH-dependent cationic polyelectrolyte having at least one functional group, selected from amino, imino, amine oxide, phosphine oxide and pyridine-N-oxide groups, preferably amine oxide, phosphine oxide, pyridine-N-oxide and pyridinium groups.

10. The method according to one or more of the preceding claims, **characterized in that** boundary layer complexes, in particular in the form of polycation/polyanion, cationic surfactant/polyanion, polycation/anionic surfactant and/or anionic surfactant/cationic surfactant complexes, are formed in boundary layers formed between the respective coating layers, in particular the boundary layer complex being, depending on the stoichiometry, water-soluble, moderately water-soluble or water-insoluble and/or in particular the water solubility of the boundary layer complexes depending on the pH, in particular on the pH of the dispersing agent.

11. The method according to one or more of the preceding claims, **characterized in that** a plurality of coating layers, in particular of at least two and up to ten or more coating layers, is applied to the at least one organic peroxycarboxylic acid, in particular imidoperoxycarboxylic acid.

12. The method according to one or more of the preceding claims, **characterized in that** the solutions and/or dispersions containing at least one polyelectrolyte and/or at least one ionic surfactant and provided in method step (a), (b) and/or optionally (c) have a pH of at most 6, in particular a pH of from 1 to 6, preferably from 2 to 5, preferably from 3 to 4, particularly preferably of approximately 3.5.

13. The method according to one or more of the preceding claims, **characterized in that**, in method step (a), (b) and/or optionally (c), the solution and/or dispersion is applied to the peroxycarboxylic acid to be stabilized and/or to the preceding coating layer by means of a coating pan, a drum coater, a mixer, in particular by means of a spray dryer, a Wurster coater, preferably by means of spraying the solution and/or dispersion in a fluidized bed unit.

14. The method according to one or more of the preceding claims, **characterized in that** at least one complexing agent, in particular selected from the group consisting of quinoline and/or salts thereof, phosphates, alkali metal polyphosphonates, picolinic acid and dipicolinic acid, mono- or polyphosphonic acids, in particular 1-hydroxyethylidene-1,1-diphosphonic acid (HEDP), ethylenediaminetetraacetic acid (EDTA), diethylenetriaminepenta(methylenephosphonic acid) (DTPMP), azacycloheptane diphosphonate (AHP) and/or nitrilotriacetic acid (NTA), in particular for complexing heavy metal ions, is added to at least one coating layer, and/or **in that** at least one plasticizer, in particular at least one plasticizer for water-soluble polymers, preferably polyethylene glycol, glycerol, glycol or triacetin, is added to at least one coating layer.

15. The method according to one or more of the preceding claims, **characterized in that**, in method step (a), (b) and/or optionally (c), the capsule system is processed, in particular dried and/or cleaned and/or sized, in particular after the particular coating layer has been applied by means of conventional methods, in particular by freeze drying (lyophilization), evaporating the dispersing agent, preferably at a temperature of from 40 °C to 60 °C in a fluidized bed unit, ultrafiltration, dialysis or spray drying under mild conditions, or sieving.

16. The method according to one or more of the preceding claims, **characterized in that** the processing, in particular drying and/or cleaning and/or sizing, optionally carried out in method step (d), is carried out by conventional methods, in particular as specified in claim 15.

17. The method according to one or more of the preceding claims, **characterized in that** the capsule system loaded with at least one organic peroxycarboxylic acid, in particular imidoperoxycarboxylic acid, is obtained having an average size (spherical diameter) of from 20 µm to 4,000 µm, preferably 50 µm to 3,000 µm, preferably 100 µm to 2,000 µm.

18. The method according to one or more of the preceding claims, **characterized in that** the proportion of the particular coating layer of the capsule coating is from 1 to 15 wt.%, preferably from 1.5 to 10 wt.%, preferably from 2 to 10 wt.%, based on the capsule system, and/or **in that** the molecular weight of the polyelectrolyte in the particular coating layer is ≥ 1,000, preferably ≥ 10,000, preferably ≥ 15,000, and/or **in that** the content of organic peroxycarboxylic acid, in particular imidoperoxycarboxylic acid, preferably PAP, is ≥ 30 wt.%, preferably ≥ 40 wt.%, preferably ≥ 50 wt.%, based on the capsule system.

19. The method according to one or more of the preceding claims, **characterized in that** the capsule system obtained in method step (b), (c) and/or optionally (d), together with additional ingredients, is formulated to form a washing or cleaning agent, in particular a liquid washing or cleaning agent, in particular the washing or cleaning agent:
- comprising at least substantially no halide ions, in particular no chloride ions, and the amount of halide ions, in particular chloride ions being at most 500 ppm, preferably at most 100 ppm, particularly preferably at most 30 ppm; and/or
- having a pH of at most 7, in particular a pH of from 3.5 to 7, preferably from 4.0 to 6.5, particularly preferably from 4.5 to 6, very particularly preferably of approximately 5; and/or
- containing at least one complexing agent, in particular selected from the group consisting of quinoline and/or salts thereof, alkali metal polyphosphonates, picolinic acid and dipicolinic acid, mono- or polyphosphonic acids, in particular 1-hydroxyethylidene-1,1-diphosphonic acid (HEDP), ethylenediaminetetraacetic acid (EDTA), diethylenetriaminepenta(methylenephosphonic acid) (DTPMP), azacycloheptane diphosphonate (AHP), nitrilotriacetic acid (NTA), citrate and/or short-chain dicarboxylic acids, in particular for complexing heavy metal ions; and/or
- optionally containing at least one water-miscible solvent, preferably glycerol, having a low solvent power for the organic peroxycarboxylic acid, in particular imidoperoxycarboxylic acid; and/or
- optionally containing at least one enzyme, in particular at least one catalase and/or at least one peroxidase, preferably at least one catalase, and/or at least one antioxidant.

20. The method according to one of claims 1 to 19 for preparing a multilayer capsule system loaded with at least one imidoperoxycarboxylic acid, preferably PAP.

21. The method according to one of claims 1 to 20 for stabilizing peroxycarboxylic acids, in particular imidoperoxycarboxylic acids, preferably PAP, and/or for increasing storage life of peroxycarboxylic acids, in particular imidoperoxycarboxylic acids, preferably PAP.

22. A capsule system, which can be obtained by the method according to claims 1 to 21.

23. Surfactant dispersions, in particular aqueous dispersions, containing the capsule system according to claim 22.

24. The use of the capsule system according to claim 22 and/or the dispersions according to claim 23 for washing and cleaning agents, in particular liquid washing and cleaning agent compositions, dental care products, hair dyes or for decolorizing or bleaching compositions for technical applications.

25. A washing and cleaning agent, in particular liquid washing and cleaning agent compositions, dental care products, hair dyes or decolorizing or bleaching compositions for technical applications, containing the capsule system according to claim 22 and/or dispersions according to claim 23.

## Revendications

1. Procédé de fabrication d'un système de capsules multicouches remplies d'au moins un peracide organique, en particulier d'un imidoperacide, **caractérisé en ce qu'**au moins deux couches d'enrobage successives différentes l'une de l'autre, respectivement à base d'au moins un polyélectrolyte et/ou un tensioactif ionique, sont appliquées sur un peracide organique, en particulier un imidoperacide, présent sous forme de particules solides, de sorte qu'il en résulte un système de capsules contenant au moins un peracide organique, en particulier un imidoperacide, dans un enrobage capsulaire constitué d'au moins deux couches d'enrobage, comprenant les étapes de procédé suivantes :
(a) Application d'au moins une solution et/ou d'une dispersion contenant au moins un premier polyélectrolyte et/ou au moins un premier tensioactif ionique (I) sur un peracide organique, en particulier un imidoperacide, présent sous forme de particules solides, de sorte que le peracide organique soit entièrement enrobé ou resp. recouvert d'une première couche d'enrobage à base du premier polyélectrolyte et/ou du premier tensioactif ionique (I), puis
(b) Application d'au moins une solution et/ou d'une dispersion contenant au moins un deuxième polyélectrolyte et/ou au moins un deuxième tensioactif ionique (II) sur la première couche d'enrobage obtenue à l'étape de procédé (a), le deuxième polyélectrolyte et/ou le deuxième tensioactif ionique (II) étant différent et chargé inversement du premier polyélectrolyte et/ou du premier tensioactif ionique (I), de sorte que soit appliquée une deuxième couche d'enrobage à base du deuxième polyélectrolyte et/ou du deuxième tensioactif ionique (II) sur la première couche d'enrobage obtenue à l'étape de procédé (a), la deuxième couche d'enrobage enrobant ou resp. recouvrant entièrement la première couche d'enrobage et les deux couches d'enrobage étant en contact direct l'une avec l'autre ; puis
(c) Application éventuelle d'au moins une autre couche d'enrobage, en particulière d'une troisième, quatrième, etc. couche d'enrobage, les polyélectrolytes et/ou tensioactifs ioniques constituant la ou les couches d'enrobage étant choisis de telle sorte que respectivement des couches d'enrobage immédiatement contiguës comprennent ou soient constituées de polyélectrolytes différents les uns des autres, en particulier de charges opposées, et/ou de tensioactifs ioniques de charges opposées ; enfin
(d) Traitement éventuel, en particulier séchage et/ou purification et/ou tri, en particulier tamisage du système de capsules obtenu,
où le polyélectrolyte et/ou le tensioactif ionique de la première couche d'enrobage obtenue à l'étape de procédé (a) et éventuellement de la troisième, cinquième, etc. couche d'enrobage obtenue à l'étape de procédé (c) présentent une charge nette positive, et la première couche d'enrobage est formée par un tensioactif positif ou un polyélectrolyte positif, de préférence par un tensioactif positif, et la troisième, cinquième, etc. couche d'enrobage éventuellement présente est formée d'un polyélectrolyte cationique (polycation), et le polyélectrolyte et/ou le tensioactif ionique de la deuxième couche d'enrobage obtenue à l'étape de procédé (b) et éventuellement de la quatrième, sixième, etc. couche d'enrobage obtenue à l'étape de procédé (c) présentent une charge nette négative, en particulier le polyélectrolyte de la deuxième et éventuellement de la quatrième, sixième, etc. couche d'enrobage est un polyélectrolyte anionique (polyanion), et/ou la couche d'enrobage la plus externe présente ou se compose préférentiellement d'un polyélectrolyte anionique.

2. Procédé selon la revendication 1, **caractérisé en ce que** le peroxyde organique est choisi parmi des mono et diperacides organiques, en particulier le peracide dodécandioïque, ou préférentiellement des imidoperacides, en particulier le peracide 6-phtalimidocaproïque, (peracide 6-phtalimidohexanoïque, PAP), et/ou **en ce que** le peracide présente sous pression atmosphérique un point de fusion supérieur à 25°C, en particulier supérieur à 35°C, de préférence supérieur à 45°C, préférentiellement supérieur à 50°C, tout particulièrement supérieur à 100°C.

3. Procédé selon une ou plusieurs des revendications précédentes, **caractérisé en ce que** la première couche d'enrobage comprend préférentiellement au moins un tensioactif cationique et/ou en ce que la deuxième couche d'enrobage et l'éventuelle troisième, quatrième, etc. couche d'enrobage comprennent au moins un tensioactif ou un mélange d'au moins un polyélectrolyte avec au moins un tensioactif ionique, où l'au moins un polyélectrolyte et l'au moins un tensioactif ionique présentent une charge nette similaire, et comprennent tout à fait préférentiellement au moins un polyélectrolyte.

4. Procédé selon une ou plusieurs des revendications précédentes, **caractérisé en ce que** le tensioactif cationique, en particulier celui de la première couche d'enrobage, est choisi parmi des sels d'ammonium quaternaire, dont le résidu correspond à la formule générale R¹R²R³N⁺, où les résidus R¹, R² et R³, identiques ou différents, présentent un atome d'hydrogène ou un résidu alkyle, alcényle ou alcynyle linéaire ou ramifié avec 1 à 40 atomes de carbone, en particulier 1 à 25 atomes de carbone, et/ou **en ce que** le tensioactif cationique et choisi en particulier dans le groupe des tensioactifs alkyldiméthylammoniums, sels de N-alkylpyridinium et esterquats, en particulier où le tensioactif cationique ne contient pas d'ions halogénures, en particulier pas d'ions chlorures, ou au moins est essentiellement exempt d'halogènes, en particulier exempt de chlore, et est choisi préférentiellement parmi des composés méthylsulfates, sulfates, phosphates, tosylates ou cumène sulfonates.

5. Procédé selon une ou plusieurs des revendications précédentes, **caractérisé en ce que** le polyélectrolyte cationique, en particulier celui de la première couche d'enrobage, comprend au moins un groupe fonctionnel choisi parmi les groupes amine quaternaire, imine et imidazole, et/ou **en ce que** le polyélectrolyte cationique, en particulier celui de la première couche d'enrobage, est choisi parmi des oxydes d'amine, N-oxydes de pyridines, de préférence la N-oxo-polyvinylpyridine, en particulier où le polyélectrolyte cationique ne contient pas d'ions halogénures, en particulier pas d'ions chlorures, ou au moins est essentiellement exempt d'halogènes, en particulier exempt de chlore.

6. Procédé selon une ou plusieurs des revendications précédentes, **caractérisé en ce que** le polyélectrolyte anionique, en particulier celui de la deuxième, quatrième, etc. couche d'enrobage, est un polyélectrolyte anionique synthétique, choisi dans le groupe des acides sulfoniques polymères, en particulier des acides polystyrène sulfoniques et de leurs sels (partiels) ; de préférence des polyacides carboxyliques et de leurs sels (partiels), comme en particulier l'acide polyacrylique, l'acide polymaléique et leurs copolymères, et/ou **en ce que** le polyélectrolyte anionique est un polymère sulfoné analogue à un polymère et/ou **en ce que** le polyélectrolyte anionique est un polymère anionique naturel choisi dans le groupe de l'acide alginique, du xanthane et/ou de polymères naturels dérivés, comme la carboxyméthylcellulose.

7. Procédé selon une ou plusieurs des revendications précédentes, **caractérisé en ce que** le polyélectrolyte anionique, en particulier celui de la deuxième, quatrième, etc. couche d'enrobage, est un polymère anionique naturel, en particulier choisi dans le groupe de l'alginate, de la carboxyméthylamylose, de la carboxyméthylcellulose, du carboxyméthyldextrane, du carraghénane, du sulfate de cellulose, du sulfate de chondroïtine, du sulfate de chitosan, du sulfate de dextrane, de la gomme arabique, de la gomme de guar, de la gomme gellane, de l'héparine, de l'acide hyaluronique, de la pectine, du xanthane et de protéines anioniques, et/ou **en ce que** le polyélectrolyte est un polyélectrolyte anionique synthétique, choisi en particulier dans le groupe des polyacrylates, des polyaminoacides anioniques et de leurs copolymères, du polymaléinate, du polyméthacrylate, du polystyrène sulfate, du polystyrène sulfonate, du polyphosphate de vinyle, du polyphosphonate de vinyle, du polysulfate de vinyle, du polyacrylamideméthylpropanesulfonate, du polylactate, du poly-(butadiène/maléinate), du poly-(éthylène/maléinate), du poly-(éthacrylate/acrylate) et du poly-(glycérine/méthacrylate).

8. Procédé selon une ou plusieurs des revendications précédentes, **caractérisé en ce que** le polyélectrolyte cationique, en particulier celui de la troisième, cinquième, etc. couche d'enrobage, est un polymère cationique naturel ou un polyélectrolyte cationique naturel modifié, choisi en particulier dans le groupe du chitosan, des dextranes modifiés, comme les dextranes modifiés diéthylaminoéthyle, de l'hydroxyméthylcellulosetriméthylamine, du lysozyme, de la polylysine, du sulfate de protamine, l'hydroxyéthylcellulosetriméthylamine et des protéines cationiques, et/ou **en ce que** le polyélectrolyte cationique est un polyélectrolyte cationique synthétique, choisi en particulier dans le groupe de la polyallylamine, des hydrosels de polyallylamine, des polyamines, des sels de polyvinylbenzyltriméthylammonium, du polybrène, des sels de polydiallyldiméthylammonium, de la polyéthylènimine, de la polyimidazoline, de la polyvinylamine, de la polyvinylpyridine, des sels de poly-(acryamide/méthacryloxypropyltriméthylammonium), des poly-(sels de diallyldiméthylammonium/N-isopropylacrylamide), du poly-(acrylate de diméthylaminoéthyl/acrylamide), du polyméthacrylate de diméthylaminoéthyle, de la polydiméthylaminoépichlorhydrine, de la polyéthyléniminoépichlorhydrine, des sels de polyméthacryloxyéthyltriméthylammonium, des sels d'hydroxypropylméthacryloxyéthyldiméthylammonium, du poly-(méthyldiéthylaminoéthylméthacrylate/acrylamide), de la polyméthylguanidine, des sels de polyméthylvinylpyridinium, du poly-(vinylpyrrolidone/méthacrylate de diméthylaminnoéthyle) et des sels de polyvinylméthylpyridinium, en particulier où le polyélectrolyte cationique ne contient essentiellement pas d'ions halogénures, en particulier pas d'ions chlorures, ou au moins est essentiellement exempt d'halogènes, en particulier exempt de chlore.

9. Procédé selon une ou plusieurs des revendications précédentes, **caractérisé en ce que** le tensioactif cationique, en particulier celui de la première, troisième, cinquième, etc. couche d'enrobage, est un tensioactif cationique dépendant du pH, en particulier du pH du solvant de dispersion, en particulier où le tensioactif cationique dépendant du pH soit présent au moins essentiellement sous forme protonée et/ou cationique à un pH de préférence inférieur à 7, en particulier inférieur à 6,5, préférentiellement inférieur à 6, et/ou où le tensioactif cationique dépendant du pH soit présent sous forme au moins essentiellement déprotonée ou électriquement neutre à un pH neutre ou alcalin, et/ou **en ce que** le polyélectrolyte cationique est un polyélectrolyte cationique dépendant du pH, en particulier du pH du solvant de dispersion, en particulier où le polyélectrolyte cationique dépendant du pH soit présent au moins essentiellement sous forme protonée et/ou cationique à un pH de préférence inférieur à 7, en particulier inférieur à 6,5, préférentiellement inférieur à 6, et/ou où le polyélectrolyte cationique dépendant du pH présente au moins un groupe fonctionnel choisi parmi les groupes amino, imino, oxyde d'amine, oxyde de phosphine et N-oxo-pyridine, de préférence oxyde d'amine, oxyde de phosphine, N-oxo-pyridine et pyridinium.

10. Procédé selon une ou plusieurs des revendications précédentes, **caractérisé en ce que** sont formés des complexes de couche limite dans les couches limites formées entre les couches d'enrobage respectives, en particulier sous forme de complexes polycation/polyanion, tensioactif cationique/polyanion, polycation/tensioactif anionique et/ou tensioactif anionique/tensioactif cationique, en particulier où les complexes de couche limite sont solubles dans l'eau, modérément solubles dans l'eau ou insolubles dans l'eau en fonction de la stoechiométrie, et/ou où la solubilité dans l'eau des complexes de couche limite dépend du pH, en particulier du pH du solvant de dispersion.

11. Procédé selon une ou plusieurs des revendications précédentes, **caractérisé en ce que** plusieurs couches d'enrobage, en particulier d'au moins deux à jusqu'à dix couches d'enrobage ou plus sont appliquées sur l'au moins un peracide organique, en particulier l'au moins un imidoperacide.

12. Procédé selon une ou plusieurs des revendications précédentes, **caractérisé en ce que** les solutions contenant au moins un polyélectrolyte et/ou au moins un tensioactif ionique préparées à l'étape de procédé (a), (b) et/ou éventuellement (c) présentent un pH de maximum 6, en particulier un pH de 1 à 6, de préférence de 2 à 5, préférentiellement de 3 à 4, particulièrement préférentiellement d'environ 3,5.

13. Procédé selon une ou plusieurs des revendications précédentes, **caractérisé en ce qu'**à l'étape de procédé (a), (b) et/ou éventuellement (c), l'application de la solution et/ou de la dispersion sur le peracide à stabiliser et/ou sur la couche d'enrobage préalable se fait par dragéificateur, enrobeuse, mélangeur, en particulier par sécheur à pulvérisation, enrobeuse Wurster, de préférence par pulvérisation de la solution et/ou de la dispersion dans un dispositif à lit fluidisé.

14. Procédé selon une ou plusieurs des revendications précédentes, **caractérisé en ce qu'**on ajoute à au moins une couche d'enrobage un complexant, choisi en particulier dans le groupe de la quinoléine et/ou de ses sels, des phosphates, des polyphosphonates de métal alcalin, de l'acide picolinique et de l'acide dipicolinique, des acides mono ou polyphosphiques, en particulier l'acide 1-hydroxyéthylidèn-1,1-diphosphonique (HEDP), l'acide éthylènediamine tétraacétique (EDTA), l'acide diéthylènetriamine penta(méthylènephosphonique) (DTPMP), l'azacycloheptanediphosphonate (AHP) et/ou l'acide nitrilotriacétique (NTA), en particulier pour complexer des ions de métaux lourds, et/ou **en ce qu'**on ajoute à au moins une couche d'enrobage au moins un adoucissant, en particulier au moins un adoucissant pour polymères solubles dans l'eau, de préférence l'éthylène glycol, la glycérine, le glycol ou la triacétine.

15. Procédé selon une ou plusieurs des revendications précédentes, **caractérisé en ce qu'**à l'étape de procédé (a), (b) et/ou éventuellement (c), on a un traitement, en particulier un séchage et/ou une purification et/ou un tri du système de capsules, en particulier après application de la couche d'enrobage correspondante, par des procédés traditionnels, en particulier par séchage à froid (lyophilisation), vaporisation du solvant de dispersion, de préférence à une température de 40°C à 60°C dans un dispositif à lit fluidisé, ultrafiltration, dialyse ou séchage par pulvérisation en conditions douces, tamisage.

16. Procédé selon une ou plusieurs des revendications précédentes, **caractérisé en ce qu'**à l'étape de procédé (d), on a éventuellement un traitement, en particulier un séchage et/ou une purification et/ou un tri, par des procédés traditionnels, en particulier comme défini à la revendication 15.

17. Procédé selon une ou plusieurs des revendications précédentes, **caractérisé en ce qu'**on obtient le système de capsules chargé d'au moins un peracide organique, en particulier un imidoperacide, avec une granulométrie moyenne (diamètre de sphère) de 20 µm à 4 000 µm, de préférence de 50 µm à 3 000 µm, préférentiellement de 100 µm à 2 000 µm.

18. Procédé selon une ou plusieurs des revendications précédentes, **caractérisé en ce que** la proportion de chaque couche d'enrobage des couches des capsules est de 1 à 15 % en poids, de préférence de 1,5 à 10 % en poids, préférentiellement de 2 à 10 % en poids, rapporté au système de capsules, et/ou **en ce que** le poids moléculaire du polyélectrolyte dans la couche d'enrobage correspondante est ≥ 1 000, de préférence ≥ 10 000, préférentiellement ≥ 15 000, et/ou **en ce que** la teneur en peracide organique, en particulier en imidoperacide, de préférence PAP, est ≥ 30 % en poids, de préférence ≥ 40 % en poids, préférentiellement ≥ 50 % en poids, rapporté au système de capsules.

19. Procédé selon une ou plusieurs des revendications précédentes, **caractérisé en ce que** le système de capsules obtenu à l'étape de procédé (b), (c) et/ou éventuellement (d), associé à d'autres ingrédients, est formulé en un produit de lavage ou de nettoyage, en particulier en un produit de lavage ou de nettoyage liquide, en particulier où le produit de lavage ou de nettoyage :
- ne présente essentiellement pas d'ions halogénures, en particulier pas d'ions chlorures, et la quantité en ions halogénures, en particulier en ions chlorures, est de maximum 500 ppm, de préférence maximum 100 ppm, particulièrement préférentiellement maximum 30 ppm ; et/ou
- présente un pH de maximum 7, en particulier un pH de 3,5 à 7, de préférence de 4,0 à 6,5, particulièrement préférentiellement de 4,5, à 6, tout particulièrement préférentiellement d'environ 5; et/ou
- contient au moins un complexant, choisi en particulier dans le groupe de la quinoléine et de ses sels, des polyphosphonates de métal alcalin, de l'acide picolinique et de l'acide dipicolinique, des acides mono ou polyphosphiques, en particulier l'acide 1-hydroxyéthylidèn-1,1-diphosphonique (HEDP), l'acide éthylènediamine tétraacétique (EDTA), l'acide diéthylènetriamine penta(méthylènephosphonique) (DTPMP), l'azacycloheptanediphosphonate (AHP), l'acide nitrilotriacétique (NTA), le citrate et/ou des acides dicarboxyliques à courte chaîne, en particulier pour complexer des ions de métaux lourds ; et/ou
- contient éventuellement au moins un solvant miscible à l'eau avec une faible capacité de solubilisation du peracide organique, en particulier d'un imidoperacide, de préférence de la glycérine ; et/ou
- contient éventuellement au moins un enzyme, en particulier au moins une catalase et/ou au moins une peroxydase, de préférence au moins une catalase, et/ou au moins un antioxydant.

20. Procédé selon l'une des revendications 1 à 19 pour la fabrication d'un système de capsules multicouches remplies d'au moins un imidoperacide, de préférence PAP.

21. Procédé selon l'une des revendications 1 à 20 pour la stabilisation de peracides organiques, en particulier d'imidoperacides, de préférence du PAP, et/ou pour l'augmentation de la durée de stockage de peracides organiques, en particulier d'imidoperacides, de préférence du PAP.

22. Système de capsules qui s'obtient selon le procédé selon les revendications 1 à 21.

23. Dispersions par tensioactifs, en particulier dispersions aqueuses, contenant le système de capsules selon la revendication 22.

24. Utilisation du système de capsules selon la revendication 22 et/ou des dispersions selon la revendication 23 pour un produit de lavage et de nettoyage, en particulier pour des compositions de produit de lavage et de nettoyage liquides, produits de soin dentaire, produits de coloration capillaire, ou pour des compositions de décoloration ou resp. de blanchiment pour applications techniques.

25. Produit de lavage et de nettoyage, en particulier compositions de produit de lavage et de nettoyage liquides, produits de soin dentaire, produits de coloration capillaire, ou compositions de décoloration ou resp. de blanchiment pour applications techniques, contenant le système de capsules selon la revendication 22 et/ou des dispersions selon la revendication 23.
